# EUROPEAN PATENT SPECIFICATION

(11) **EP 3 336 178 B1**
(45) Date of publication and mention of the grant of the patent: **18.03.2020**
(21) Application number: 16834853.0
(22) Date of filing: 18.05.2016
(51) Int. Cl.: C12N 5/10, C12M 1/00, C12M 3/00, C12N 5/071, C07K 14/50, C07K 14/78, C12N 5/073, C12N 5/074

(54) **METHOD FOR CULTURING PLURIPOTENT STEM CELLS, METHOD FOR MANUFACTURING CULTURE VESSEL, CULTURE VESSEL, AND SCAFFOLD MATERIAL FOR CULTURING CELLS**
VERFAHREN ZUR KULTIVIERUNG PLURIPOTENTER STAMMZELLEN, VERFAHREN ZUR HERSTELLUNG EINES KULTURGEFÄSSES, KULTURGEFÄSS UND GERÜSTMATERIAL ZUR ZELLKULTIVIERUNG
PROCÉDÉ DE CULTURE DE CELLULES SOUCHES PLURIPOTENTES, PROCÉDÉ DE FABRICATION D'UN RÉCIPIENT DE CULTURE, RÉCIPIENT DE CULTURE ET MATÉRIAU D'ÉCHAFAUDAGE POUR LA CULTURE DE CELLULES

(30) Priority: 13.08.2015 JP 2015159967
(43) Date of publication of application: 20.06.2018
(73) Proprietor: Fujifilm Corporation, Minato-ku Tokyo 106-8620 (JP)
(72) Inventor: MURAKAMI, Yuta, Kanagawa 258-8577 (JP)
(74) Representative: Hoffmann Eitle
(86) International application number: PCT/JP2016/064757
(87) International publication number: WO 2017/026156

(56) References cited:
- EP-A1- 1 923 078
- WO-A1-2013/164970
- WO-A1-2014/117146
- WO-A1-2015/037622
- WO-A1-2015/064661
- JP-A- H01 179 685
- JP-A- 2005 176 630
- JP-A- 2007 068 884
- JP-A- 2007 508 816
- JP-A- 2014 100 141
- JP-A- 2015 037 433
- UNDERWOOD P ANNE ET AL: "Effects of base material, plasma proteins and FGF2 on endothelial cell adhesion and growth.", JOURNAL OF BIOMATERIALS SCIENCE. POLYMER EDITION 2002, vol. 13, no. 8, 2002, pages 845-862, XP002782295, ISSN: 0920-5063
- PROWSE ANDREW B J ET AL: "Long term culture of human embryonic stem cells on recombinant vitronectin in ascorbate free media.", BIOMATERIALS NOV 2010, vol. 31, no. 32, November 2010 (2010-11), pages 8281-8288, XP002782296, ISSN: 1878-5905
- HINO, RIKA ET AL.: 'The generation of germline transgenic silkworms for the production of biologically active recombinant fusion proteins of fibroin and human basic fibroblast growth factor' BIOMATERIALS vol. 27, 2006, pages 5715 - 5724, XP027951130
- JUN KOBAYASHI ET AL. SAIBO ZOSHOKU O SOKUSHIN SURU TAMENO JISEDAIGATA ONDO OTOSEI BAIYOZARA vol. 33, 30 November 2013, pages 144 - 145, XP009504200
- STEMCELL TECHNOLOGIES INC: 'HUMAN PLURIPOTENT STEM CELL RESEARCH TSSRTM2 MAINTENANCE MEDIUM FOR HUMAN ES ANDIPS CELLS' PRODUCT INFORMATION SHEET, [Online] 01 January 2015, pages 1 - 2, XP055460027 Retrieved from the Internet: <URL:HTTP://WWW.VERITASTK.CO.JP/ATTACHED/35 79/29930_PIS_1_0_1.PDF>

## Description

### BACKGROUND OF THE INVENTION

### 1. Field of the Invention

The present invention relates to a method for culturing pluripotent stem cells, a method for manufacturing a culture vessel, a culture vessel, and a scaffold material for culturing cells.

### 2. Description of the Related Art

In order to maintain the undifferentiated state of pluripotent stem cells and allow stable proliferation thereof, it is considered that basic fibroblast growth factor (bFGF) is required to be present in a medium. bFGF is disclosed in, for example, JP5717311B and JP4613069B, and is used as a supplement in a liquid medium for culturing the pluripotent stem cells. bFGF is included in serum or a serum substitute, and even by adding the serum or the serum substitute to the liquid medium, bFGF can be used as a supplement in a liquid medium for culturing the pluripotent stem cells.

In the related art, in the culture of pluripotent stem cells, a mouse fibroblast is used as a feeder cell. However, from the viewpoint of preventing a heterologous infectious disease, a scaffold for culturing pluripotent stem cells has been suggested as a substitute for the mouse fibroblast. For example, WO2013/164970A and Biomaterials, 2010, Vol. 31, No. 32, pp. 8281-8288 suggest a recombinant polypeptide including a partial sequence of human vitronectin.

WO 2014/117146 A1 relates to growth matrices for stem cell propagation in vitro and in tissue regeneration.

### SUMMARY OF THE INVENTION

It is known that bFGF is a substance having low stability in a liquid medium, and is gradually degraded, denatured, or deactivated in a liquid medium. Therefore, in order to cause stable proliferation of pluripotent stem cells while maintaining the undifferentiated state thereof, it is necessary to add a large amount of bFGF to a liquid medium, frequently replace the medium with a liquid medium containing bFGF, or replenish the liquid medium with bFGF during the culturing.

Embodiments of the present invention were made under the above circumstances.

An object of the embodiments of the present invention is to provide a method for culturing pluripotent stem cells, a method for manufacturing a culture vessel, a culture vessel, and a scaffold material for culturing cells by which the undifferentiated state of pluripotent stem cells is maintained, and pluripotent stem cells stably proliferate, even in a liquid medium which does not substantially contain bFGF or in a liquid medium in which the bFGF content is lower than a normal bFGF content. The problem of the embodiments is to achieve these objects.

Specific means for solving the above problem include the following aspects.
[A1] A method for culturing pluripotent stem cells comprising: a culturing process, which is performed in a liquid medium which does not substantially contain bFGF: the process of bringing pluripotent stem cells into contact with a polypeptide which has cell adhesion activity and includes a first domain for binding bFGF and a second domain for adhering to a culture vessel and culturing the pluripotent stem cells, in which bFGF is bound to the first domain, and the second domain is adhered to the culture vessel.
[A2] The method for culturing pluripotent stem cells according to [A1], in which the first domain has a negative charge.
[A3] The method for culturing pluripotent stem cells according to [A1] or [A2], in which the first domain includes any one of the following sequence A-1, sequence A-2, and sequence A-3, and the second domain includes any one of the following sequence B-1, sequence B-2, and sequence B-3.
   Sequence A-1: an amino acid sequence represented by SEQ ID No: 2
   Sequence A-2: an amino acid sequence which shares identity of equal to or higher than 80% with the amino acid sequence represented by SEQ ID No: 2 and has the ability to bind bFGF
   Sequence A-3: an amino acid sequence which is obtained by addition, substitution, or deletion of one or several amino acid residues in the amino acid sequence represented by SEQ ID No: 2 and has the ability to bind bFGF
   Sequence B-1: an amino acid sequence represented by SEQ ID No: 3
   Sequence B-2: an amino acid sequence which shares identity of equal to or higher than 80% with the amino acid sequence represented by SEQ ID No: 3 and has the ability to adhere to the culture vessel
   Sequence B-3: an amino acid sequence which is obtained by addition, substitution, or deletion of one or several amino acid residues in the amino acid sequence represented by SEQ ID No: 3 and has the ability to adhere to the culture vessel
[A4] The method for culturing pluripotent stem cells according to any one of [A1] to [A3], in which the polypeptide further includes an RGD motif.
[A5] The method for culturing pluripotent stem cells according to any one of [A1] to [A4], in which the polypeptide includes the first domain at the terminal of the molecule.
[A6] The method for culturing pluripotent stem cells according to any one of [A1] to [A5], in which the polypeptide is a recombinant based on an amino acid sequence of natural human vitronectin.
[A7] The method for culturing pluripotent stem cells according to any one of [A1] to [A6], in which the polypeptide consists of 80 to 500 amino acid residues.
[A8] The method for culturing pluripotent stem cells according to any one of [A1] to [A7], in which a GRAVY value of the polypeptide is -2.0 to -0.95.
[A9] The method for culturing pluripotent stem cells according to any one of [A1] to [A8], further comprising, before the culturing process: an adhering process of bringing the culture vessel into contact with the polypeptide which has cell adhesion activity and includes the first domain for binding bFGF and the second domain for adhering to the culture vessel and adhering the second domain to the culture vessel; and a binding process of bringing the polypeptide into contact with a solution containing bFGF and allowing bFGF to be bound to the first domain.
[A10] The method for culturing pluripotent stem cells according to [A9], further comprising, before the culturing process: a removing process of removing the solution which is brought into contact with the polypeptide.
[A11] The method for culturing pluripotent stem cells according to [A9] or [A10], in which a content of bFGF in the solution is in a range of 100 ng/mL to 1000 ng/mL.
[A12] A method for culturing pluripotent stem cells, comprising: a culturing process, which is performed in a liquid medium: the process of bringing pluripotent stem cells into contact with bFGF which is directly or indirectly adhered to a culture vessel and a polypeptide which has cell adhesion activity and is adhered to the culture vessel and culturing the pluripotent stem cells.
[A13] The method for culturing pluripotent stem cells according to [A12], in which bFGF is indirectly adhered to the culture vessel by being bound to a negatively charged material which is adhered to the culture vessel.
[A14] The method for culturing pluripotent stem cells according to [A12], in which bFGF is directly adhered to the culture vessel by being bound to the culture vessel which has a negative charge.
[A15] The method for culturing pluripotent stem cells according to any one of [A 12] to [A14], in which the liquid medium does not substantially contain bFGF.
[A16] The method for culturing pluripotent stem cells according to any one of [A1] to [A15], in which the pluripotent stem cells are human iPS cells or human ES cells.
[B1] A method for manufacturing a culture vessel by which a culture vessel including a polypeptide that has cell adhesion activity and has bFGF bound thereto is manufactured, the method comprising: an adhering process of bringing the culture vessel into contact with the polypeptide which has cell adhesion activity and includes a first domain for binding bFGF and a second domain for adhering to the culture vessel and adhering the second domain to the culture vessel; and a binding process of bringing the polypeptide into contact with a solution containing bFGF and allowing bFGF to be bound to the first domain.
[B2] The method for manufacturing a culture vessel according to [B1], further comprising: a removing process of removing the solution which is brought into contact with the polypeptide.
[B3] The method for manufacturing a culture vessel according to [B1] or [B2], in which a content of bFGF in the solution is in a range of 100 ng/mL to 1000 ng/mL.
[B4] The method for manufacturing a culture vessel according to any one of [B1] to [B3], further comprising: a drying process of drying the polypeptide which is adhered to the culture vessel and has bFGF bound thereto.
[B5] The method for manufacturing a culture vessel according to any one of [B1] to [B4], further comprising: an impregnation process of impregnating the polypeptide which is adhered to the culture vessel and has bFGF bound thereto with a liquid which does not substantially contain bFGF.
[B6] The method for manufacturing a culture vessel according to any one of [B1] to [B5], in which the first domain includes any one of the sequence A-1, the sequence A-2, and the sequence A-3, and the second domain includes any one of the sequence B-1, the sequence B-2, and the sequence B-3.
[B7] The method for manufacturing a culture vessel according to any one of [B1] to [B6], in which the polypeptide further includes an RGD motif.
[B8] The method for manufacturing a culture vessel according to any one of [B1] to [B7], in which the polypeptide includes the first domain at the terminal of the molecule.
[B9] The method for manufacturing a culture vessel according to any one of [B1] to [B8], in which the polypeptide is a recombinant based on an amino acid sequence of natural human vitronectin.
[B10] The method for manufacturing a culture vessel according to any one of [B1] to [B9], in which the polypeptide consists of 80 to 500 amino acid residues.
[B11] The method for manufacturing a culture vessel according to any one of [B1] to [B10], in which the culture vessel is for culturing pluripotent stem cells.
[B12] The method for manufacturing a culture vessel according to [B11], in which the pluripotent stem cells are human iPS cells or human ES cells.
[C1] A culture vessel comprising: a polypeptide which has cell adhesion activity and includes a first domain for binding bFGF and a second domain for adhering to a culture vessel, in which bFGF is bound to the first domain and the second domain is adhered to the culture vessel.
[C2] The culture vessel according to [C1], in which the polypeptide is in a dry state.
[C3] The culture vessel according to [C1], in which the polypeptide is in a wet state in which a liquid that does not substantially contain bFGF is retained.
[C4] The culture vessel according to any one of [C1] to [C3], in which the first domain includes any one of the sequence A-1, the sequence A-2, and the sequence A-3, and the second domain includes any one of the sequence B-1, the sequence B-2, and the sequence B-3.
[C5] The culture vessel according to any one of [C1] to [C4], in which the polypeptide further includes an RGD motif.
[C6] The culture vessel according to any one of [C1] to [C5], in which the polypeptide includes the first domain at the terminal of the molecule.
[C7] The culture vessel according to any one of [C1] to [C6], in which the polypeptide is a recombinant based on an amino acid sequence of natural human vitronectin.
[C8] The culture vessel according to any one of [C1] to [C7], in which the polypeptide consists of 80 to 500 amino acid residues.
[C9] The culture vessel according to any one of [C1] to [C8], which is for culturing pluripotent stem cells.
[C10] The culture vessel according to [C9], in which the pluripotent stem cells are human iPS cells or human ES cells.
[D1] A scaffold material for culturing cells, comprising: a polypeptide which has cell adhesion activity and includes a first domain for binding bFGF and a second domain for adhering to a culture vessel, in which bFGF is bound to the first domain.
[D2] The scaffold material for culturing cells according to [D1], which is in a dry state.
[D3] The scaffold material for culturing cells according to [D1], which is in a wet state in which a liquid that does not substantially contain bFGF is retained.
[D4] The scaffold material for culturing cells according to any one of [D1] to [D3], in which the first domain includes any one of the sequence A-1, the sequence A-2, and the sequence A-3, and the second domain includes any one of the sequence B-1, the sequence B-2, and the sequence B-3.
[D5] The scaffold material for culturing cells according to any one of [D1] to [D4], in which the polypeptide further includes an RGD motif.
[D6] The scaffold material for culturing cells according to any one of [D1] to [D5], in which the polypeptide includes the first domain at the terminal of the molecule.
[D7] The scaffold material for culturing cells according to any one of [D1] to [D6], in which the polypeptide is a recombinant based on an amino acid sequence of natural human vitronectin.
[D8] The scaffold material for culturing cells according to any one of [D1] to [D7], in which the polypeptide consists of 80 to 500 amino acid residues.
[D9] The scaffold material for culturing cells according to any one of [D1] to [D8], which is for culturing pluripotent stem cells.
[D10] The scaffold material for culturing cells according to [D9], in which the pluripotent stem cells are human iPS cells or human ES cells.

According to the present disclosure, a method for culturing pluripotent stem cells, a method for manufacturing a culture vessel, a culture vessel, and a scaffold material for culturing cells by which the undifferentiated state of pluripotent stem cells is maintained, and pluripotent stem cells stably proliferate, even in a liquid medium which does not substantially contain bFGF or in a liquid medium in which the bFGF content is lower than a normal bFGF content, are provided.

### BRIEF DESCRIPTION OF THE DRAWINGS

Fig. 1 is a graph showing the result of determining the amounts of bFGF by measuring absorbance in Example 3.
Fig. 2 shows optical microscope images of human iPS cells cultured in Example 4.
Fig. 3 shows graphs showing the results of comparative assessment of the gene expression level in Example 6.

### DESCRIPTION OF THE PREFERRED EMBODIMENTS

Hereinafter, embodiments of the present invention will be described. These descriptions and Examples are provided to exemplify the present invention, and are not intended to limit the scope of the present invention.

In the present specification, numerical ranges expressed using "to" indicate a range including the numerical values before and after "to" as the lower limit value and the upper limit value, respectively.

In a case where a process cannot be clearly distinguished from other processes, the process is included in the term "process" in the present specification, in addition to independent processes, as long as the intended object of the process is achieved.

In a case where the amount of each component in a composition is indicated in the present specification, the amount indicates the total amount of plural kinds of substances that exist in the composition, in a case where plural kinds of substances corresponding to each component exist in the composition, unless otherwise particularly specified.

In the present specification, there are cases where an amino acid residue in an amino acid sequence is indicated using one-letter codes (for example, a glycine residue is indicated as "G") or three-letter codes (for example, a glycine residue is indicated as "Gly"), which is well-known in the present technical field.

The term "identity" in relation to an amino acid sequence in the present specification refers to a value calculated using the BLAST package (refer to Ausubel et al., 1999, Short Protocols in Molecular Biology, 4th Ed-Chapter 18). For example, sharing identity of equal to or higher than 80% with an amino acid sequence represented by SEQ ID No: 3 means that the Max Identity value in the BLAST is equal to or higher than 80%.

In the present specification, an expression such as "a corresponding amino acid residue" used for a specific amino acid residue in an amino acid sequence refers to, in a case where two or more amino acid sequences to be compared are aligned such that there is a maximum number of amino acid residues identical to each other using a method well-known in the present technical field while considering insertion, deletion, and substitution, an amino acid residue in an amino acid sequence of which the position coincides with the position of a specific amino acid residue in the other amino acid sequence which serves as a standard.

In the present disclosure, the expression that a liquid medium or a liquid "does not substantially contain bFGF" means that the amount of bFGF which is contained as a component of a liquid medium or a liquid does not exceed the minimum amount that is required for maintaining the undifferentiated state of pluripotent stem cells and for stably proliferating the pluripotent stem cells. Specifically, a concentration of bFGF in a liquid medium or a liquid is preferably lower than 5 ng/ml, more preferably lower than 1 ng/ml, and even more preferably equal to or lower than the detection limit.

In the present disclosure, there are cases where a polypeptide which has cell adhesion activity and includes a first domain for binding bFGF and a second domain for adhering to a culture vessel is referred to as a "specific polypeptide", and in a case where bFGF is bound to the first domain of the specific polypeptide, the specific polypeptide is referred to as a "specific polypeptide/bFGF complex".

The present disclosure provides a first embodiment and a second embodiment as methods for culturing pluripotent stem cells. Hereinafter, these two embodiments will be described in order.

### <Method for Culturing Pluripotent Stem Cells: First Embodiment>

A first embodiment is a method for culturing pluripotent stem cells including a culturing process, which is performed in a liquid medium which does not substantially contain bFGF: the process of bringing pluripotent stem cells into contact with a polypeptide having 80 to 250 amino acid residues which has cell adhesion activity and has a first domain, as defined in the claims, for binding bFGF and a second domain, as defined in the claims, for adhering to a culture vessel and culturing the pluripotent stem cells, in which bFGF is bound to the first domain, and the second domain is adhered to the culture vessel.

That is, in the first embodiment, a specific polypeptide/bFGF complex which is in the state of being adhered to the culture vessel is brought into contact with the pluripotent stem cells, and the pluripotent stem cells are cultured. In the first embodiment, the specific polypeptide/bFGF complex serves as a scaffold for proliferation of the pluripotent stem cells.

In the first embodiment, degradation, denaturation, or inactivation of bFGF can be suppressed by the binding of bFGF to the specific polypeptide. In addition, since the pluripotent stem cells are cultured while being in contact with the specific polypeptide/bFGF complex, the pluripotent stem cells are under the action of bFGF in the specific polypeptide/bFGF complex, and thus the undifferentiated state of the cells can be maintained and the cells can stably proliferate.

However, according to the first embodiment, the pluripotent stem cells can be cultured in the liquid medium that does not substantially contain bFGF, and it is not necessary to frequently replace the medium with a liquid medium containing bFGF and/or add bFGF to a liquid medium in the culture, for the purpose of replenishing the culture environment with bFGF.

Hereinafter, the first embodiment will be described in detail.

### [Specific Polypeptide]

The amino acid sequence and the number of amino acid residues in a specific polypeptide is 80 to 250, and the specific polypeptide has cell adhesion activity and includes a first domain, as defined in the claims, for binding bFGF and a second domain, as defined in the claims, for adhering to a culture vessel.

In the present disclosure, the expressions "binds bFGF" and "has the ability to bind bFGF" used for a polypeptide or the amino acid sequence thereof mean that the polypeptide binds or can bind bFGF through general protein-protein interactions (hydrogen bonding, ionic bonding, bonding due to an electrostatic interaction, a hydrophobic interaction, and Van der Waals forces, and the like). In the present disclosure, whether a polypeptide has the ability to bind bFGF or not is determined by the following method.

The polypeptide is brought into contact with bFGF at a concentration of 100 ng/ml. For example, after the polypeptide is adhered to a culture vessel, a solution in which the bFGF concentration is 100 ng/ml (for example, a bFGF aqueous solution, or Essential 8 medium) is poured into the culture vessel. In a case where the polypeptide exists in a liquid, bFGF may be added to the polypeptide solution such that a concentration of bFGF becomes 100 ng/ml. Then, the solution is left to stand at 37°C for 24 hours, and after being left to stand for 24 hours, Enzyme-Linked Immunosorbent Assay (ELISA) is performed using an antibody to bFGF, and detection of bFGF is carried out. In a case where the amount of bFGF detected is significantly higher than that in a solution in which the bFGF concentration is 100 ng/ml (for example, a bFGF aqueous solution or Essential 8 medium; since bFGF does not form a complex with other compounds in this solution, bFGF changes into a form that cannot be detected by ELISA in a case of being left to stand at 37°C for 24 hours), it is determined that the polypeptide has the ability to bind bFGF.

In the present disclosure, the expressions "adheres to a culture vessel" and "has the ability to adhere to a culture vessel" used for a polypeptide or the amino acid sequence thereof mean that the polypeptide adheres to or can adhere to a cell culture surface of the culture vessel (hereinafter, may be referred to as a "culture surface") without being subjected to a chemical treatment. Whether a polypeptide has the ability to adhere to the culture surface of a culture vessel or not is determined by the following method.

A polypeptide-containing solution is introduced into a polystyrene culture vessel of which the inner surface has been subjected to a plasma treatment, such that the density becomes 200 pmol/cm², and the solution is left to stand at 37°C for 2 hours. After being left to stand for 2 hours, the culture vessel is washed twice with a phosphate buffer solution (pH 7.4), and then whether the polypeptide remains on the culture surface at a density of 10 pmol/cm² or higher is determined. The amount of the polypeptide remaining on the culture surface is determined by ELISA using an antibody that recognizes the polypeptide or by High Performance Liquid Chromatography (HPLC) in which the adhered polypeptide is hydrolyzed, and the amount of amino acid, thus generated, is measured.

In the present disclosure, cell adhesion activity of a polypeptide or the amino acid sequence thereof is adhesion activity with respect to a cell which is the culture target, and can be confirmed by a known method. For example, cells are seeded in a culture vessel of which the culture surface is coated with the polypeptide and incubated, and the number of cells that adhere to the culture surface is counted, so as to confirm the cell adhesion activity. Specific methods are described in, for example, Methods in Enzymology 82: 803-831, 1982 (Ruoslahti, E. et al.) and Nature 352: 438-441, 1991 (Williams, D. A. et al.).

The specific polypeptide is preferably a polypeptide of which the first domain has a negative charge. Since bFGF has a positive charge, the first domain electrostatically binds bFGF in a case where the first domain has a negative charge. Whether the polypeptide or a portion of the polypeptide has a positive charge or a negative charge depends on the pH environment of the polypeptide or a portion of the polypeptide. The expression that the first domain "has a negative charge" means that the domain has a negative charge in a cell culture, for example, in a liquid medium of pH 7.4. Examples of the embodiment in which the first domain has a negative charge in the cell culture include an embodiment in which the isoelectric point of the first domain is 6 or lower, and an embodiment in which the isoelectric point of the first domain is 1 to 6 is preferable, and an embodiment in which the isoelectric point of the first domain is 2 to 5 is more preferable.

Examples of the specific polypeptide desclosed herein include a natural protein such as vitronectin, fibronectin, type I collagen, and type IV collagen, a recombinant protein designed based on the amino acid sequences of these natural proteins, and a mixture thereof. The specific polypeptide can be used alone, or two or more kinds thereof may be used in combination.

As the vitronectin, human vitronectin is preferable, and specifically, a polypeptide of 459 amino acid residues represented by SEQ ID No: 1 is preferable. Natural human vitronectin produced in a human body has been confirmed to be a glycoprotein in which sugar chains are bonded to some of the amino acids.

Vitronectin represented by SEQ ID No: 1 includes a first domain for binding bFGF and a second domain for adhering to a culture vessel and has the ability to bind bFGF and the ability to adhere to the culture vessel. The vitronectin represented by SEQ ID No: 1 further includes a domain that exhibits cell adhesion activity and adheres to a cell that is a culture target.

The specific polypeptide is preferably a recombinant designed based on an amino acid sequence of natural human vitronectin (SEQ ID No: 1), from the viewpoint of reducing the concern of contamination by an antigenic substance and an infectious agent. Hereinafter, the recombinant may be referred to as a "recombinant vitronectin".

The specific polypeptide is a polypeptide having, as the first domain, a domain including any one of the following sequences A-1 to A-3, and, as the second domain, a domain including any one of the following sequences B-1 to B-3.
Sequence A-1: an amino acid sequence represented by SEQ ID No: 2
Sequence A-2: an amino acid sequence which shares identity of equal to or higher than 80% with the amino acid sequence represented by SEQ ID No: 2 and has the ability to bind bFGF
Sequence A-3: an amino acid sequence which is obtained by addition, substitution, or deletion of one or several amino acid residues in the amino acid sequence represented by SEQ ID No: 2 and has the ability to bind bFGF
   SEQ ID No: 2
   DQESCKGRCTEGFNVDKKCQCDELCSYYQSCCTDYTAECKPQ
Sequence B-1: an amino acid sequence represented by SEQ ID No: 3
Sequence B-2: an amino acid sequence which shares identity of equal to or higher than 80% with the amino acid sequence represented by SEQ ID No: 3 and has the ability to adhere to the culture vessel
Sequence B-3: an amino acid sequence which is obtained by addition, substitution, or deletion of one or several amino acid residues in the amino acid sequence represented by SEQ ID No: 3 and has the ability to adhere to the culture vessel
   SEQ ID No: 3
   PRPSLAKKQRFRHRNRKGYRSQRGHSRGRNQN

The amino acid sequence represented by SEQ ID No: 2 consists of 42 amino acid residues which are the 1^{st} to 42^{nd} amino acid residues in SEQ ID No: 1 and corresponds to a somatomedin B-like domain. In the present disclosure, the amino acid sequence represented by SEQ ID No: 2 and an amino acid sequence which shares identity with the amino acid sequence represented by SEQ ID No: 2 may be referred to as an "SMB domain". The SMB domain has the ability to bind bFGF.

The amino acid sequence represented by SEQ ID No: 3 consists of 32 amino acid residues which are the 342^{nd} to 373^{rd} amino acid residues in SEQ ID No: 1, corresponds to a heparin-binding domain, and is included in a part of hemopexin-like domain II. In the present disclosure, the amino acid sequence represented by SEQ ID No: 3 and an amino acid sequence which shares identity with the amino acid sequence represented by SEQ ID No: 3 may be referred to as a "heparin-binding domain". The heparin-binding domain has the ability to adhere to the culture vessel. The heparin-binding domain exhibits the effect of securing hydrophilicity of the polypeptide, thus inhibiting hydrophobic aggregation of the polypeptide.

The sequence A-2 is an amino acid sequence which shares identity of equal to or higher than 80% with the amino acid sequence represented by SEQ ID No: 2, and is an amino acid sequence which preferably shares identity of equal to or higher than 85%, more preferably equal to or higher than 90%, and even more preferably equal to or higher than 95%, with the amino acid sequence represented by SEQ ID No: 2.

The sequence A-3 is an amino acid sequence which is obtained by addition, substitution, or deletion of one or several amino acid residues in the amino acid sequence represented by SEQ ID No: 2, and is preferably an amino acid sequence which is obtained by addition, substitution, or deletion of 1 to 8 amino acids, and more preferably, 1 to 4 amino acids in the amino acid sequence represented by SEQ ID No: 2.

The sequence B-2 is an amino acid sequence which shares identity of equal to or higher than 80% with the amino acid sequence represented by SEQ ID No: 3, and is an amino acid sequence which preferably shares identity of equal to or higher than 85%, more preferably equal to or higher than 90%, and even more preferably equal to or higher than 95%, with the amino acid sequence represented by SEQ ID No: 3.

The sequence B-3 is an amino acid sequence which is obtained by addition, substitution, or deletion of one or several amino acid residues in the amino acid sequence represented by SEQ ID No: 3, and is preferably an amino acid sequence which is obtained by addition, substitution, or deletion of 1 to 6 amino acids, and more preferably, 1 to 3 amino acids in the amino acid sequence represented by SEQ ID No: 3.

It is preferable that the specific polypeptide further includes an RGD motif. An RGD motif consists of Arg-Gly-Asp (arginine-glycine-aspartic acid) and is an amino acid sequence having cell adhesion activity. The RGD motif in natural human vitronectin is the 45^{th} to 47^{th} sequence in SEQ ID No: 1.

It is preferable that the specific polypeptide exposes the RGD motif on the surface of the molecule, from the viewpoint of cell adhesion activity. Therefore, the amino acid sequences immediately before and immediately after the RGD motif in the specific polypeptide is preferably designed based on the amino acid sequence of a natural protein (for example, natural human vitronectin) which has cell adhesion activity due to an RGD motif. From this viewpoint, one embodiment of the specific polypeptide includes at least one of (a) or (b) immediately before or immediately after the RGD motif, and another embodiment of the specific polypeptide includes both (a) and (b) immediately before and immediately after the RGD motif.
(a) The 43^{rd} and 44^{th} amino acid sequence in SEQ ID No: 1
(b) The 48^{th} to 55^{th} amino acid sequence in SEQ ID No: 1

It is preferable that (a) is positioned immediately before the RGD motif, and (b) is positioned immediately after the RGD motif. (a) and (b) may be amino acid sequences which are obtained by addition, substitution, or deletion of one or two amino acids respectively, within a range that does not impair the cell adhesion activity of the RGD motif.

Relative positions of the first domain and the second domain in the specific polypeptide are not particularly limited. In one embodiment of the specific polypeptide, the first domain is positioned on the N-terminal side of the second domain.

In a case where the specific polypeptide includes the RGD motif, the position of the RGD motif in the specific polypeptide is not particularly limited. In one embodiment of the specific polypeptide, the RGD motif is positioned between the first domain and the second domain.

It is preferable that the specific polypeptide includes the first domain at the terminal of the molecule, from the viewpoint of enhancing the ability to bind bFGF and from the viewpoint of exposing bound bFGF on the surface of the molecule. In one embodiment of the specific polypeptide, the first domain is positioned at the N-terminal.

A domain including the first domain and the RGD motif in the specific polypeptide preferably consists of the 1^{st} to 55^{th} amino acid sequence in SEQ ID No: 1 or an amino acid sequence which shares identity with this amino acid sequence. That is, the domain including the first domain and the RGD motif in the specific polypeptide is preferably a domain consisting of any one of the following sequences C-1 to C-3.
Sequence C-1: an amino acid sequence represented by SEQ ID No: 4
Sequence C-2: an amino acid sequence which shares identity of equal to or higher than 80% with the amino acid sequence represented by SEQ ID No: 4, has the ability to bind bFGF, and includes the RGD motif
Sequence C-3: an amino acid sequence which is obtained by addition, substitution, or deletion of one or several amino acid residues in the amino acid sequence represented by SEQ ID No: 4, has the ability to bind bFGF, and includes the RGD motif

The sequence C-2 is an amino acid sequence which shares identity of equal to or higher than 80% with the amino acid sequence represented by SEQ ID No: 4, and is an amino acid sequence which preferably shares identity of equal to or higher than 85%, more preferably equal to or higher than 90%, and even more preferably equal to or higher than 95%, with the amino acid sequence represented by SEQ ID No: 4.

The sequence C-3 is an amino acid sequence which is obtained by addition, substitution, or deletion of one or several amino acid residues in the amino acid sequence represented by SEQ ID No: 4, and is preferably an amino acid sequence which is obtained by addition, substitution, or deletion of 1 to 10 amino acids, and more preferably, 1 to 5 amino acids in the amino acid sequence represented by SEQ ID No: 4.

In one embodiment of the specific polypeptide, the domain consisting of any one of the sequences C-1 to C-3 is positioned at the N-terminal. The specific polypeptide of this embodiment is preferable, from the viewpoint of enhancing the ability to bind bFGF, from the viewpoint of exposing bound bFGF on the surface of the molecule, and from the viewpoint of exposing the RGD motif on the surface of the molecule.

The specific polypeptide may include other amino acid sequences besides the first domain, the second domain, and the RGD motif, and the other amino acid sequences are preferably partial sequences of natural human vitronectin (SEQ ID No: 1). By the inclusion of a partial sequence of natural human vitronectin, the specific polypeptide can have the properties that resemble the properties of human vitronectin.

It is preferable that the specific polypeptide includes the partial sequence of natural human vitronectin (SEQ ID No: 1) immediately before or immediately after the second domain, from the viewpoint of the ability to bind bFGF, the ability to adhere to the culture vessel, cell adhesion activity, or the suppression of aggregation. Examples of the partial sequence include the following sequences D-1 to D-3 and sequences E-1 to E-3.
Sequence D-1: the 269^{th} to 341^{st} amino acid sequence (sequence d-1) of the amino acid sequence represented by SEQ ID No: 1 or a partial amino acid sequence thereof
Sequence D-2: an amino acid sequence which shares identity of equal to or higher than 80% with the amino acid sequence of the sequence d-1 or a partial amino acid sequence thereof
Sequence D-3: an amino acid sequence which is obtained by addition, substitution, or deletion of one or several amino acid residues in the amino acid sequence of the sequence d-1 or a partial amino acid sequence thereof
Sequence E-1: the 374^{th} to 459^{th} amino acid sequence (sequence e-1) of the amino acid sequence represented by SEQ ID No: 1 or a partial amino acid sequence thereof
Sequence E-2: an amino acid sequence which shares identity of equal to or higher than 80% with the amino acid sequence of the sequence e-1 or a partial amino acid sequence thereof
Sequence E-3: an amino acid sequence which is obtained by addition, substitution, or deletion of one or several amino acid residues in the amino acid sequence of the sequence e-1 or a partial amino acid sequence thereof

The term "partial amino acid sequence" used above refers to an amino acid sequence consisting of three or more consecutive amino acid residues that is a part of an amino acid sequence in a predetermined range.

The sequence D-2 is a sequence which shares identity of equal to or higher than 80% with the amino acid sequence of the sequence d-1 or a partial amino acid sequence thereof, and for example, is an amino acid sequence which shares identity of equal to or higher than 85%, equal to or higher than 90%, or equal to or higher than 95%, with the amino acid sequence of the sequence d-1 or a partial amino acid sequence thereof.

The sequence D-3 is an amino acid sequence which is obtained by addition, substitution, or deletion of one or several amino acid residues in the amino acid sequence of the sequence d-1 or a partial amino acid sequence thereof, and for example, is an amino acid sequence which is obtained by addition, substitution, or deletion of 1 to 20, 1 to 10, or 1 to 5 amino acids in the amino acid sequence of the sequence d-1 or a partial amino acid sequence thereof.

The sequence E-2 is a sequence which shares identity of equal to or higher than 80% with the amino acid sequence of the sequence e-1 or a partial amino acid sequence thereof, and for example, is an amino acid sequence which shares identity of equal to or higher than 85%, equal to or higher than 90%, or equal to or higher than 95%, with the amino acid sequence of the sequence e-1 or a partial amino acid sequence thereof.

The sequence E-3 is an amino acid sequence which is obtained by addition, substitution, or deletion of one or several amino acid residues in the amino acid sequence of the sequence e-1 or a partial amino acid sequence thereof, and for example, is an amino acid sequence which is obtained by addition, substitution, or deletion of 1 to 20, 1 to 10, or 1 to 5 amino acids in the amino acid sequence of the sequence e-1 or a partial amino acid sequence thereof.

In a case where the specific polypeptide is recombinant vitronectin and includes other amino acid sequences besides the first domain, the second domain, and the RGD motif, the other amino acid sequences may have an amino acid residue other than a cysteine residue at the cysteine residue position in the amino acid sequence represented by SEQ ID No: 1. This allows the prevention of the intramolecular cross-linking or intermolecular cross-linking formation by cysteine residues, which is preferable. Examples of the amino acid residue that substitutes for the cysteine residue include a serine residue, an alanine residue, a glycine residue, and the like. Among these, a serine residue and an alanine residue are preferable, from the viewpoint of having a structure similar to that of a cysteine residue.

In a case where the specific polypeptide is recombinant vitronectin, the specific polypeptide may not include the 56^{th} to 268^{th} amino acid residues in the amino acid sequence represented by SEQ ID No: 1. It is considered that this domain contributes little to the performance required for the specific polypeptide in the present disclosure.

The specific polypeptide may include additional amino acid residues other than those described so far, within a range that does not impair cell adhesion activity, the ability to bind bFGF, and the ability to adhere to the culture vessel. Examples of such amino acid residues include an additional sequence added for the preparation of the polypeptide by a recombination technology, and specific examples thereof include a methionine residue at the N-terminal, a tag sequence (for example, a GST tag, a FLAG tag, a His tag, and the like), a linker sequence between each domain (for example, a sequence formed by repetition of GGS, GGGS, GGGGS, or the like), and the like.

It is preferable that the specific polypeptide disclosed herein consists of 80 to 500 amino acid residues. In a case where the specific polypeptide consists of 80 or more amino acid residues, the ability to bind bFGF, the ability to adhere to the culture vessel, cell adhesion activity, and cell proliferation activity are satisfactorily exhibited. From this viewpoint, the number of amino acid residues in the specific polypeptide is preferably 80 or more, more preferably 85 or more, even more preferably 90 or more, and still more preferably 100 or more. On the other hand, in a case where the specific polypeptide consists of 500 or less amino acid residues, association, cross-linking, or aggregation between proteins do not easily occur. From this viewpoint, the number of amino acid residues in the specific polypeptide is preferably 500 or less, more preferably 480 or less, even more preferably 460 or less, still more preferably 400 or less, still more preferably 300 or less, still more preferably 250 or less, still more preferably 200 or less, still more preferably 180 or less, and still more preferably 150 or less. Any combinations of these upper limit values and lower limit values may be adopted. For example, the specific polypeptide consists of 80 to 400 amino acid residues, 80 to 300 amino acid residues, 80 to 250 amino acid residues, 80 to 200 amino acid residues, 80 to 150 amino acid residues, or 100 to 150 amino acid residues.

A Grand Average of Hydropathy (GRAVY) value of the specific polypeptide is preferably -2.0 to -0.95, from the viewpoint of excellent ability to adhere to the culture vessel and from the viewpoint of inhibiting hydrophobic aggregation. A high GRAVY value (Kyte J., Doolittle R. F. (1982), J. Mol. Biol, 157: 105-132) indicates that hydrophobicity of a polypeptide is high. In a case where the GRAVY value is -0.95 or lower, hydrophobic aggregation does not easily occur. From this viewpoint, the GRAVY value of the specific polypeptide is preferably -0.95 or lower, more preferably -0.975 or lower, even more preferably -1.0 or lower, and still more preferably -1.10 or lower. On the other hand, in a case where the GRAVY value is -2.0 or higher, cell proliferation activity and the ability to adhere to the culture vessel are easily exhibited, and this effect tends to be heightened as the GRAVY value increases. From this viewpoint, the GRAVY value of the specific polypeptide is preferably -2.0 or higher, more preferably -1.70 or higher, and even more preferably -1.60 or higher. The GRAVY value of the specific polypeptide can be adjusted by increasing or decreasing the proportion of hydrophobic amino acids (for example, Trp, Tyr, Phe, Leu, Ile, Val, and Met) in the amino acid sequence, increasing or decreasing the number of amino acid residues, or the like.

As the specific polypeptide of the disclosure, a polypeptide having the following (1) as the domain including the first domain and the RGD motif and the following (2) as the second domain, further having at least one of the following (3) or (4), and consisting of 80 to 500 amino acid residues is preferable, from the viewpoint of the ability to bind bFGF, the ability to adhere to the culture vessel, cell adhesion activity, cell proliferation activity, and undifferentiation maintaining properties.
(1) A domain consisting of any one of the 1^{st} to 55^{th} amino acid sequence in SEQ ID No: 1 (that is, the sequence C-1: the amino acid sequence represented by SEQ ID No: 4) and an amino acid sequence which shares identity with this amino acid sequence (that is, the sequence C-2 or the sequence C-3)
(2) A domain consisting of any one of the 342^{nd} to 373^{rd} amino acid sequence in SEQ ID No: 1 (that is, the sequence B-1: the amino acid sequence represented by SEQ ID No: 3) and an amino acid sequence which shares identity with this amino acid sequence (that is, the sequence B-2 or the sequence B-3)
(3) A domain consisting of any one of the 269^{th} to 341^{st} amino acid sequence in SEQ ID No: 1 or a partial amino acid sequence thereof (that is, the sequence D-1) and an amino acid sequence which shares identity with these amino acid sequences (that is, the sequence D-2 or the sequence D-3)
(4) A domain consisting of any one of the 374^{th} to 459^{th} amino acid sequence in SEQ ID No: 1 or a partial amino acid sequence thereof (that is, the sequence E-1) and an amino acid sequence which shares identity with these amino acid sequences (that is, the sequence E-2 or the sequence E-3)

In one embodiment of the specific polypeptide, (1) to (4) are arranged in the order of (1), (3), (2) and (4) from the N-terminal side.

Specific examples of the amino acid sequence of the specific polypeptide are shown in Table 1. The SMB domain, the RGD motif, and the heparin-binding domain in the amino acid sequence are underlined.

**[Table 1]**

| SEQ ID No. | Amino acid sequence | Number of amino acid residues | Corresponding position in SEQ ID No: 1 |
|---|---|---|---|
| 5 | | 87 | 1-55, 342-373 |
| 6 | | 107 | 1-55, 322-373 |
| 7 | | 117 | 1-55, 312-373 |
| 8 | | 127 | 1-55, 302-373 |
| 9 | | 142 | 1-55, 270-277, 295-373, C274S |
| 10 | | 159 | 1-55, 270-373, C274S |
| 11 | | 165 | 1-55, 270-379, C274S |
| 12 | | 175 | 1-55, 270-389, C274S |
| 13 | | 185 | 1-55, 270-399, C274S |
| 14 | | 245 | 1-55, 270-459, C274S, C411S, C453S |
| 15 | | 246 | 1-55, 269-459 |

Specific examples of the amino acid sequence of the specific polypeptide include an amino acid sequence which is obtained by adding a methionine residue to the N-terminal of an amino acid sequence represented by any of SEQ ID Nos: 5 to 15. The methionine residue at the N-terminal is added, for example, in a case where the polypeptide is prepared by a recombination technology.

The specific polypeptide can be manufactured by a gene recombination technology known to those skilled in the art. For example, the specific polypeptide can be manufactured according to the method described in WO2013/164970A. Specifically, a gene that encodes an amino acid sequence designed based on the amino acid sequence of natural human vitronectin is acquired and incorporated into an expression vector to prepare a recombinant expression vector, which is introduced into a suitable host so as to prepare a transformant. The obtained transformant is cultured in a suitable medium, to thereby produce a culture containing recombinant vitronectin, and the recombinant vitronectin is collected from the culture, whereby the recombinant vitronectin is obtained as the specific polypeptide.

### [Liquid Medium]

The liquid medium used in the first embodiment is not particularly limited, as long as the medium does not substantially contain bFGF. The liquid medium may contain components suitable to the type of pluripotent stem cells that become the culture target. As the liquid medium, a liquid medium which is a known basal medium for mammalian cells (preferably a basal medium for human cells, and more preferably a basal medium for human stem cells) and does not contain bFGF; and a liquid medium obtained by removing bFGF from a known basal medium for mammalian cells (preferably a basal medium for human cells, and more preferably a basal medium for human stem cells) are preferable. Examples of the basal medium for mammalian cells that can be applied in the present disclosure include Dulbecco's Modified Eagle's Medium (DMEM), Dulbecco's Modified Eagle Medium: Nutrient Mixture F-12 (DMEM: F-12), Eagle's minimal essential medium (EMEM), Minimum Essential Medium Alpha (MEMα), Basal Medium Eagle (BME), Roswell Park Memorial Institute 1640 Medium (RPMI 1640 Medium), E8 base medium (Nature Protocols 7: 2029-2040, 2012), Skeletal Muscle Cell Basal Medium (SkBM), MCDB104, MCDB153, 199, L15, mTeSRI, TeSR2, and the like.

Various components that are generally added to a liquid medium, for example, an antibiotic substance such as penicillin or streptomycin; a vitamin or a vitamin derivative such as ascorbic acid or retinoic acid; a sugar source such as glucose; an amino acid; an inorganic salt such as sodium selenite or sodium chloride; a protein such as transferrin; a hormone such as insulin; a cell growth factor such as transforming growth factor-β (TGF-β) or epidermal growth factor (EGF); a differentiation inhibitory factor; an antioxidant such as 2-mercaptoethanol or dithiothreitol; and the like may be added to the liquid medium at usual concentrations. The components may be replenished to the liquid medium in the culture of pluripotent stem cells in order to maintain the concentrations of the components within a desired range throughout the entire culture period.

It is preferable that the liquid medium does not contain serum and a serum substitute, from the viewpoint of suppressing incorporation of bFGF into the liquid medium and from the viewpoint of suppressing contamination of the pluripotent stem cells by foreign matters such as an antigenic substance and an infectious agent.

A pH of the liquid medium is, for example, pH 7.0 to 8.0, and is preferably pH 7.3 to 7.4.

### [Culture Condition]

As a culture condition in the first embodiment, a general condition may be adopted. For example, culturing in an incubator at a temperature of 37°C and 5% (v/v) CO₂ concentration is adopted. In a case of passaging the pluripotent stem cells, a general passage method may be adopted.

A seeding density of the pluripotent stem cells is not particularly limited, and a general condition may be adopted. For example, the seeding density may be approximately 1×10³ cells/cm² to 1×10⁵ cells/cm². In addition, cell masses each having a size of 10 µm to 100 µm may be seeded at a seeding density of approximately 1 cell mass/cm² to 5 cell masses/cm².

In first embodiment, by adhering the specific polypeptide/bFGF complex to the culture vessel, the specific polypeptide/bFGF complex is not easily lost from the culture vessel in a case where the liquid medium is replaced. In addition, degradation, denaturation, or inactivation of bFGF in the specific polypeptide/bFGF complex is suppressed by the binding of bFGF to the specific polypeptide. Therefore, according to the first embodiment, the culturing of the pluripotent stem cells can be continued while replacing the liquid medium which does not substantially contain bFGF within the same culture vessel.

In the first embodiment, liquid media used in a series of cultures may not have the same composition. In the first embodiment, culturing may be continued while substituting the liquid medium with a liquid medium having a different composition, as long as the pluripotent stem cells can be maintained in an undifferentiated state.

In first embodiment, it is preferable that feeder cells are not used, from the viewpoint of suppressing contamination of the pluripotent stem cells by foreign matters such as an antigenic substance and an infectious agent. In the first embodiment, since the specific polypeptide/bFGF complex adhered to the culture vessel functions as a scaffold for the proliferation of the pluripotent stem cells, feeder cells are not required.

### [Pluripotent Stem Cells]

Cells of the first embodiment that are the culture target are pluripotent stem cells. The pluripotent stem cells are undifferentiated cells that have "self-renewal ability" that allows cells to proliferate while maintaining the undifferentiated state and "multipotency" that allows differentiation of the cells into all three germ layers. Examples of the pluripotent stem cells include induced pluripotent stem cells (iPS cells), embryonic stem cells (ES cells), embryonic germ cells (EG cells), embryonal carcinoma cells (EC cells), multipotent adult progenitor cells (MAP cells), adult pluripotent stem cells (APS cells), multi-lineage differentiating stress enduring cells (Muse cells), and the like. As the pluripotent stem cells of the present disclosure that are the culture target, human iPS cells or human ES cells are preferable. iPS cells include the cells described in Nature 448: 313-317 (2007), and Cell 126: 663-676 (2006), or cells that are similar to these cells.

Whether the pluripotent stem cells have maintained the undifferentiated state or not can be confirmed by a known method. Examples of the method include a method in which the expression of an undifferentiation marker is confirmed by flow cytometry or immunostaining, confirming the formation of a teratoma in a case of being subcutaneously injected into an immunodeficient mouse, and the like.

The pluripotent stem cells may be feeder cell-dependent cells, but feeder cell-independent cells are preferable. The feeder cell-independent cells can be screened by, for example, performing passage operations several times under the absence of feeder cells, using a culture vessel of the present disclosure which will be described later.

### [Culture Vessel]

The culture vessel in the first embodiment includes, the specific polypeptide/bFGF complex adhered to the culture vessel as the scaffold for the proliferation of the cells, at least on the culture surface for performing the culturing of cells.

Examples of a main body of the culture vessel used in the first embodiment include all known culture vessels used for culturing cells in the present technical field. Materials of the main body are not particularly limited, and examples of the materials include a resin (for example, polystyrene, polycarbonate, polyester, and an acrylonitrile-butadiene-styrene resin), glass, a porous filter, a hollow fiber, ceramic, and the like. Shapes of the main body are not particularly limited as well, and examples of the shapes include a multi-well plate, a Petri dish, a plate for microarray, a tube, a flask, a roller bottle, a bag, and the like. It is preferable that the main body of the culture vessel is a polystyrene culture vessel having a culture surface on which an electric charge treatment is performed by plasma discharge, from the viewpoint of adhesiveness of the specific polypeptide and the specific polypeptide/bFGF complex. In the present specification, the electric charge treatment performed by plasma discharge is referred to as a "plasma treatment".

In the first embodiment, the culturing process may be performed using a culture vessel prepared in advance. The culture vessel used in the first embodiment can be manufactured by a method for manufacturing a culture vessel of the present disclosure which will be described later.

The first embodiment may include, before the culturing process, preparing the culture surface of the culture vessel by the following adhering process and binding process. According to this embodiment, the preparation of the culture vessel and the culturing of the pluripotent stem cells are continuously performed in order. This embodiment preferably further includes the following removing process, from the viewpoint of suppressing the incorporation of bFGF into the liquid medium.

Adhering process: a process of bringing the culture vessel into contact with the polypeptide (specific polypeptide) which has cell adhesion activity and includes the first domain for binding bFGF and the second domain for adhering to the culture vessel and adhering the second domain to the culture vessel
Binding process: a process of bringing the specific polypeptide into contact with a solution containing bFGF and allowing bFGF to be bound to the first domain
Removing process: a process of removing the solution which contains bFGF and has been brought into contact with the specific polypeptide

The adhering process, the binding process, and the removing process are the same as the respective processes described in the section of <Method for Manufacturing Culture Vessel>, and preferable aspects thereof are also the same.

### <Method for Culturing Pluripotent Stem Cells: Second Aspect>

A second aspect is a method for culturing pluripotent stem cells including a culturing process, which is performed in a liquid medium: the process of bringing pluripotent stem cells into contact bFGF which is directly or indirectly adhered to a culture vessel and a polypeptide which has cell adhesion activity and is adhered to the culture vessel and culturing the pluripotent stem cells.

In second aspect, bFGF is directly or indirectly adhered to the culture vessel, whereas the polypeptide which has cell adhesion activity is adhered to the culture vessel without forming a complex with bFGF.

In second aspect, degradation, denaturation, or inactivation of bFGF is suppressed by the direct adhesion of bFGF to the culture vessel or by the adhesion of bFGF to the culture vessel through other material. In addition, since the polypeptide which has cell adhesion activity and is adhered to the culture vessel serves as a scaffold for proliferation of the pluripotent stem cells, and the pluripotent stem cells are cultured while being in contact with bFGF, the undifferentiated state of the pluripotent stem cells is maintained, and the pluripotent stem cells stably proliferate.

Accordingly, according to the second aspect, the pluripotent stem cells can be cultured even in a liquid medium which does not substantially contain bFGF or in a liquid medium in which the bFGF content is lower than a normal bFGF content, and it is not necessary to frequently replace a liquid medium containing bFGF and/or add bFGF to the liquid medium in the culture, for the purpose of replenishing the culture environment with bFGF.

Hereinafter, the second aspect will be described in detail.

### [Polypeptide]

The polypeptide in the second aspect is not particularly limited, as long as the polypeptide has the ability to adhere to the culture vessel and cell adhesion activity. Examples of the polypeptide in the second aspect include a polypeptide which is a known scaffold material for culturing pluripotent stem cells, such as vitronectin, a polypeptide derived from vitronectin, laminin, and a polypeptide derived from laminin. One of these polypeptides can be used alone, or two or more kinds thereof may be used in combination.

An example of the polypeptide derived from vitronectin is a polypeptide which consists of an amino acid sequence obtained by deletion of at least one of a part or the entirety of the 1^{st} to 44^{th} amino acid sequence or a part or the entirety of the 379^{th} to 449^{th} amino acid sequence from an amino acid sequence of human vitronectin (SEQ ID No: 1). A more preferable example of the polypeptide derived from vitronectin is a polypeptide which consists of an amino acid sequence obtained by deletion of a part or the entirety of the 1^{st} to 44^{th} amino acid sequence and a part or the entirety of the 379^{th} to 449^{th} amino acid sequence from the amino acid sequence of human vitronectin, and an even more preferable example of the polypeptide derived from vitronectin is a polypeptide which consists of an amino acid sequence obtained by deletion of a part of the 1^{st} to 44^{th} amino acid sequence and a part of the 379^{th} to 449^{th} amino acid sequence from the amino acid sequence of human vitronectin.

Another preferable example of the polypeptide derived from vitronectin is the following polypeptide.
- A polypeptide which consists of the 62^{nd} to 478^{th} amino acid sequence in the amino acid sequence of human vitronectin (SEQ ID No: 1)
- A polypeptide which has the ability to adhere to the culture vessel and cell adhesion activity and shares identity of equal to or higher than 80% (that is, 80% to 100%; preferably 90% to 100%) with the 62^{nd} to 478^{th} amino acid sequence in the amino acid sequence of human vitronectin (SEQ ID No: 1)
- A polypeptide which has the ability to adhere to the culture vessel and cell adhesion activity, and is obtained by addition, substitution, or deletion of one or several amino acid residues (preferably 1 to 10 amino acid residues and more preferably 1 to 5 amino acid residues) in the 62^{nd} to 478^{th} amino acid sequence in the amino acid sequence of human vitronectin (SEQ ID No: 1)

Still another example of the polypeptide derived from vitronectin is VTN-N (manufactured by Life Technologies Corporation), commercially available recombinant vitronectin that can be suitably used in the second aspect.

As the polypeptide derived from laminin, a polypeptide including the E8 fragment of human laminin α5β1γ1 or the E8 fragment of human laminin α3β3γ2 is preferable. The E8 fragment of human laminin α5β1γ1 refers to a fragment of human laminin α5β1γ1 that corresponds to the E8 fragment of mouse laminin α1β1γ1, and the E8 fragment of human laminin α3β3γ2 refers to a fragment of human laminin α3β3γ2 that corresponds to mouse laminin 111E8. Specific examples of the polypeptide derived from laminin include iMatrix-511 (manufactured by Nippi. Inc.), which can be suitably used in the second embodiment.

### [bFGF Adhered to Culture Vessel]

In the second aspect, bFGF is directly or indirectly adhered to the culture vessel. In the second aspect, any one of the following is preferable: (A) bFGF is indirectly adhered to the culture vessel by electrostatically being bound to a negatively charged material which is adhered to the culture vessel and (B) bFGF is directly adhered to the culture vessel by electrostatically being bound to the culture vessel having a negative charge.

### -Aspect (A)-

Examples of the negatively charged material for the indirect adhesion of bFGF to the culture vessel that has the ability to adhere to the culture vessel include alkali-treated gelatin. Whether the material has a positive charge or a negative charge depends on the pH environment of the material. The expression that the material "is negatively charged" means that the material has a negative charge in a cell culture, for example in a liquid medium of pH 7.4. In addition, the expressions that the material "is adhered to the culture vessel" and "has the ability to adhere to the culture vessel" mean that the material is adhered to or can be adhered to a cell culture surface of the culture vessel without any chemical treatment.

The culture vessel used in the aspect (A) can be prepared by, for example, pouring a solution containing bFGF, the negatively charged material that has the ability to adhere to the culture vessel, and the polypeptide that has the ability to adhere to the culture vessel and cell adhesion activity into the culture vessel and incubating the solution. A main body of the above culture vessel is the same as the main body of the culture vessel in the first embodiment.

The aspect (A) is carried out by, for example, accommodating a liquid medium in which the bFGF content is lower than a normal bFGF content or a liquid medium which does not substantially contain bFGF in the culture vessel prepared by the method described above.

The aspect (A) may also be employed in a culture method in which the culturing of the pluripotent stem cells is initiated using the culture vessel to which the negatively charged material and the polypeptide having cell adhesion activity are adhered and a liquid medium containing bFGF. In this case, bFGF contained in the liquid medium during the initiation of the culturing is adhered to the culture vessel through the aforementioned material, and the liquid medium is either a liquid medium in which the bFGF content is lower than a normal bFGF content or a liquid medium which does not substantially contain bFGF.

### -Aspect (B)-

Examples of the culture vessel of which the culture surface for performing culturing of cells has a negative charge include a polystyrene culture vessel having a surface modified with a sulfo group and a polystyrene culture vessel having a surface modified with a carboxyl group.

The culture vessel used in the aspect (B) can be prepared by, for example, pouring a solution containing bFGF and the polypeptide having the ability to adhere to the culture vessel and cell adhesion activity into the culture vessel of which the culture surface has a negative charge and incubating the solution.

The aspect (B) is carried out by, for example, accommodating a liquid medium in which the bFGF content is lower than a normal bFGF content or a liquid medium which does not substantially contain bFGF in the culture vessel prepared by the method described above.

The aspect (B) may also be employed in a culture method in which the culturing of the pluripotent stem cells is initiated using the culture vessel to which the polypeptide having cell adhesion activity is adhered and of which the culture surface has a negative charge and a liquid medium containing bFGF. In this case, bFGF contained in the liquid medium during the initiation of the culturing is adhered to the culture vessel, and the liquid medium is either a liquid medium in which the bFGF content is lower than a normal bFGF content or a liquid medium which does not substantially contain bFGF.

The details regarding [Liquid Medium], [Culture Condition], and [Pluripotent Stem Cells] in the second aspect are the same as those described in the first embodiment, except for the descriptions that have been made so far. In the second aspect, a content of bFGF in the liquid medium is not particularly limited. As the liquid medium used in the second aspect, a liquid medium in which the bFGF content is lower than a normal bFGF content is preferable, and a liquid medium which does not substantially contain bFGF is preferable.

### <Method for Manufacturing Culture Vessel>

A method for manufacturing a culture vessel of the present disclosure includes the following adhering process and binding process. A polypeptide which has cell adhesion activity and to which bFGF is bound is disposed in a culture vessel through the following adhering process and binding process. That is, according to the method for manufacturing a culture vessel of the present disclosure, the culture vessel including a specific polypeptide/bFGF complex as a scaffold for proliferation of cells is provided.

Adhering process: a process of bringing the culture vessel into contact with a polypeptide (specific polypeptide) which has cell adhesion activity and includes a first domain for binding bFGF and a second domain for adhering to the culture vessel and adhering the second domain to the culture vessel
Binding process: a process of bringing the specific polypeptide into contact with a solution containing bFGF and allowing bFGF to be bound to the first domain

It is preferable that the method for manufacturing a culture vessel of the present disclosure further includes the following removing process, from the viewpoint of suppressing incorporation of bFGF into a liquid medium used in a case of culturing the cells.

Removing process: a process of removing the solution which contains bFGF and has been brought into contact with the specific polypeptide
The adhering process includes, for example, the following; a liquid containing the specific polypeptide (specific polypeptide-containing liquid) is poured into the culture vessel or applied onto a surface of the culture vessel and is left to stand at a temperature of 25°C to 40°C for 30 minutes to 24 hours. The specific polypeptide-containing liquid may be prepared by dissolving or dispersing the specific polypeptide in a phosphate buffer solution, a tris buffer solution, physiological saline, water, or the like. A concentration of the specific polypeptide in the specific polypeptide-containing liquid is, for example, 10 µg/mL to 1000 µg/mL. Since the specific polypeptide can adhere to the culture vessel through the second domain, a treatment of fixing the specific polypeptide onto the culture vessel through a chemical treatment is not required.

It is preferable that the specific polypeptide adheres to the surface of the culture vessel at a density in a range of 1 pmol/cm² to 1000 pmol/cm², and more preferably at a density in a range of 100 pmol/cm² to 300 pmol/cm².

The binding process includes, for example, the following; the specific polypeptide is brought into contact with the solution containing bFGF (bFGF-containing solution) and is left to stand at a temperature of 25°C to 40°C for 30 minutes to 24 hours.

The bFGF-containing solution used in the binding process may be prepared by dissolving or dispersing bFGF in a phosphate buffer solution, a tris buffer solution, physiological saline, an injection solvent, deionized water, or the like. In addition, the liquid medium containing bFGF may be used as the bFGF-containing solution. A concentration of bFGF in the bFGF-containing solution is, for example, 1 ng/mL to 10000 ng/mL, preferably 10 ng/mL to 1000 ng/mL, and more preferably 100 ng/mL to 1000 ng/mL.

It is preferable that the amount of the bFGF-containing solution used in the binding process is an amount that makes one molecule to 10 molecules of bFGF exist per 100 molecules of the specific polypeptide. As for a volume, it is preferable that the volume is equal to or larger than an amount that causes the specific polypeptide to be entirely soaked by the solution.

Any one of the adhering process and the binding process may precede the other one, or the two processes may be performed together. For example, the specific polypeptide may be adhered to the culture vessel by performing the adhering process first, and then the binding process of pouring the bFGF-containing solution into the culture vessel and leaving the solution to stand may be performed. Alternatively, the binding process may be performed by dissolving or dispersing the specific polypeptide in the bFGF-containing solution to prepare a mixture and leaving the mixture to stand, and then the adhering process may be performed by pouring the mixture into the culture vessel and leaving the mixture to stand. Furthermore, the adhering process and the binding process may be performed together on the surface of the culture vessel by dissolving or dispersing the specific polypeptide in the bFGF-containing solution to prepare a mixture and pouring the mixture into the culture vessel and leaving the mixture to stand.

The removing process includes suctioning the bFGF-containing solution used in the binding process using, for example, a glass instrument. After removing the bFGF-containing solution, the specific polypeptide/bFGF complex may be washed with a phosphate buffer solution, or the like.

The method for manufacturing a culture vessel of the present disclosure may include a drying process of drying the specific polypeptide/bFGF complex adhered to the culture vessel. By performing the drying process, the culture vessel including the specific polypeptide/bFGF complex in a dry state is obtained.

The drying process may also be performed together with the removing process. That is, the removing process of sufficiently removing the bFGF-containing solution also serves as the drying process, and through this process, the culture vessel including the specific polypeptide/bFGF complex in a dry state is obtained. Alternatively, the drying process may be a process of performing a drying treatment such as leaving the solution to stand in a low humidity environment, blast drying, and freeze-drying. These drying treatments may be performed after performing the adhering process and the binding process, or may be performed after performing the removing process.

The method for manufacturing a culture vessel of the present disclosure may include an impregnation process of impregnating the specific polypeptide/bFGF complex adhered to the culture vessel with a liquid which does not substantially contain bFGF. By performing the impregnation process, the culture vessel including the specific polypeptide/bFGF complex that is in a wet state in which a liquid that does not substantially contain bFGF is retained is obtained. The impregnation process may be performed after the adhering process and the binding process, after the removing process, or after the drying process.

Examples of the liquid that does not substantially contain bFGF include a phosphate buffer solution, a tris buffer solution, and physiological saline; a basal medium for mammalian cells, which is a liquid medium not containing bFGF; a liquid medium obtained by removing bFGF from the basal medium for mammalian cells; and the like.

The impregnation process is not necessarily required for obtaining the culture vessel including the specific polypeptide/bFGF complex that is in a wet state in which liquid that does not substantially contain bFGF is retained. For example, in a case where bFGF in the bFGF-containing solution which has been brought into contact with the specific polypeptide is bound to the specific polypeptide in the binding process, and the solution that has been brought into contact with the specific polypeptide becomes substantially free of bFGF, the culture vessel including the specific polypeptide/bFGF complex that is in a wet state in which the liquid that does not substantially contain bFGF is retained is obtained.

According to the method for manufacturing a culture vessel of the present disclosure, a culture vessel described below is obtained.

### <Culture Vessel>

A culture vessel of the present disclosure includes a polypeptide which has cell adhesion activity and includes a first domain for binding bFGF and a second domain for adhering to a culture vessel, in which bFGF is bound to the first domain and the second domain is adhered to the culture vessel. That is, the culture vessel of the present disclosure includes a specific polypeptide/bFGF complex adhered to the culture vessel as a scaffold for the proliferation of cells.

The culture vessel of the present disclosure is, as one embodiment, used in the first embodiment of the culture method of the present disclosure. That is, the culture vessel of the present disclosure accommodates a liquid medium which does not substantially contain bFGF and is used to culture pluripotent stem cells. In this case, the specific polypeptide/bFGF complex included in the culture vessel of the present disclosure serves as the scaffold for the proliferation of the pluripotent stem cells. The use of the culture vessel of the present disclosure is not limited to the use in the culturing of the pluripotent stem cells, and the culture vessel may also be used in culturing of somatic stem cells, somatic cells that constitute the living body, precursor cells of somatic cells, or the like.

In the culture vessel of the present disclosure, degradation, denaturation, or inactivation of bFGF is suppressed by binding of bFGF to the specific polypeptide. In addition, since the pluripotent stem cells are cultured while being in contact with the specific polypeptide/bFGF complex, the pluripotent stem cells are under the action of bFGF in the specific polypeptide/bFGF complex, and thus the undifferentiated state of the cells can be maintained and the cells can stably proliferate. Therefore, according to the culture vessel of the present disclosure, the pluripotent stem cells can be cultured in a liquid medium which does not substantially contain bFGF, and it is not necessary to frequently replace a liquid medium containing bFGF and/or add bFGF to the liquid medium in the culture, for the purpose of replenishing the culture environment with bFGF.

In the culture vessel of the present disclosure, by adhering the specific polypeptide/bFGF complex to the culture vessel, the specific polypeptide/bFGF complex is not easily lost from the culture vessel in a case where the liquid medium is replaced. In addition, the degradation, denaturation, or inactivation of bFGF in the specific polypeptide/bFGF complex is suppressed by the binding of bFGF to the specific polypeptide. Therefore, according to the culture vessel of the present disclosure, the culturing of the pluripotent stem cells can be continued while replacing the liquid medium which does not substantially contain bFGF within the same culture vessel.

In the culture vessel of the present disclosure, the scaffold for the proliferation of the cells (simply referred to as a "scaffold") may include other components besides the specific polypeptide/bFGF complex. Examples of the other components include the specific polypeptide itself (the specific polypeptide in a state of not binding bFGF); known extracellular matrix such as laminin and gelatin; a cell growth factor attached to these extracellular matrices; and the like.

In one embodiment of the culture vessel of the present disclosure, the specific polypeptide/bFGF complex adhered to the culture vessel, that is, the scaffold, is in a dry state. The culture vessel of this embodiment is preferable, from the viewpoint that the degradation, denaturation, or inactivation of bFGF contained in the scaffold is suppressed by the scaffold being in the dry state.

In another embodiment of the culture vessel of the present disclosure, the specific polypeptide/bFGF complex adhered to the culture vessel, that is, the scaffold, is in a wet state in which a liquid that does not substantially contain bFGF is retained. The culture vessel of this embodiment is preferable, from the viewpoint that a bond between the specific polypeptide and bFGF (that is, a bond formed by a general protein-protein interaction) is easily maintained by retaining a liquid in the scaffold.

Examples of the liquid that does not substantially contain bFGF include a phosphate buffer solution, a tris buffer solution, and physiological saline; a basal medium for mammalian cells, which is a liquid medium not containing bFGF; a liquid medium obtained by removing bFGF from the basal medium for mammalian cells; and the like.

In the present disclosure, the term "dry state" used for the scaffold for the proliferation of the cells and a scaffold material for culturing cells refers to a state in which liquid components are lost such that the amount of the residual liquid components is less than or equal to the amount that can be realized in a removing process performed by a suction treatment after a binding process and a state in which the scaffold or the scaffold material has a liquid absorption performance, without being limited to the state in which the liquid components do not exist at all. In the present disclosure, the term "wet state" used for the scaffold for the proliferation of the cells and the scaffold material for culturing cells refers to a state that is not the "dry state" and a state in which the liquid is sufficiently absorbed.

### <Scaffold Material for Culturing Cells>

According to the present disclosure, a scaffold material for culturing cells (simply referred to as a "scaffold material") is also provided. The scaffold material of the present disclosure includes a polypeptide which has cell adhesion activity and has a first domain for binding bFGF and a second domain for adhering to a culture vessel, in which bFGF is bound to the first domain. That is, the scaffold material of the present disclosure is a scaffold material that includes a specific polypeptide/bFGF complex.

The scaffold material of the present disclosure is used to provide a scaffold for proliferation of cells in the culture vessel. The scaffold that is provided in the culture vessel by the scaffold material of the present disclosure functions as a scaffold for maintaining undifferentiated state of pluripotent stem cells and allowing stable proliferation of the pluripotent stem cells. The use of the scaffold material of the present disclosure is not limited to the use in culturing of the pluripotent stem cells, and the scaffold material may also be used in culturing of somatic stem cells, somatic cells that constitute the living body, precursor cells of somatic cells, or the like.

In a case where the pluripotent stem cells are cultured using the scaffold material of the present disclosure, the undifferentiated state of the cells can be maintained and the cells can stably proliferate in a liquid medium which does not substantially contain bFGF.

The scaffold material of the present disclosure may include other components besides the specific polypeptide/bFGF complex. Examples of the other components include the specific polypeptide itself (the specific polypeptide in a state of not binding bFGF); known extracellular matrix such as laminin and gelatin; a cell growth factor attached to these extracellular matrices; and the like.

One case of the scaffold material of the present disclosure is a scaffold material in a dry state. The scaffold material of this case is preferable, from the viewpoint that degradation, denaturation, or inactivation of bFGF contained in the scaffold material is suppressed by the scaffold material being in the dry state. Examples of an aspect of using the scaffold material of this case include dissolving or dispersing the scaffold material in a liquid (for example, a phosphate buffer solution), and then pouring the solution into the culture vessel or applying the solution onto the culture vessel. As a result, a scaffold including the specific polypeptide/bFGF complex adhered to the culture vessel is formed on a culture surface of the culture vessel.

Another case of the scaffold material of the present disclosure is a scaffold material in a wet state in which a liquid that does not substantially contain bFGF is retained. The scaffold material of this case is preferable, from the viewpoint that a bond between the specific polypeptide and bFGF (that is, a bond formed by a general protein-protein interaction) is easily maintained by retaining a liquid. Examples of an aspect of using the scaffold material of this case include thinly placing or applying the scaffold material onto the culture surface of the culture vessel. As a result, a scaffold including the specific polypeptide/bFGF complex adhered to the culture vessel is formed on the culture surface of the culture vessel.

Examples of the liquid that does not substantially contain bFGF include a phosphate buffer solution, a tris buffer solution, and physiological saline; a basal medium for mammalian cells, which is a liquid medium not containing bFGF; a liquid medium obtained by removing bFGF from the basal medium for mammalian cells; and the like.

The scaffold material of the present disclosure can be manufactured by a binding process of bringing the polypeptide (specific polypeptide) which has cell adhesion activity and has the first domain for binding bFGF and the second domain for adhering to the culture vessel into contact with a solution containing bFGF (bFGF-containing solution) and allowing bFGF to be bound to the first domain. This process includes, for example, bringing the specific polypeptide into contact with the bFGF-containing solution and leaving the polypeptide in contact with the solution to stand at a temperature of 25°C to 40°C for 30 minutes to 24 hours.

The bFGF-containing solution used in the binding process may be prepared by dissolving or dispersing bFGF in a phosphate buffer solution, a tris buffer solution, physiological saline, an injection solvent, deionized water, or the like. In addition, a liquid medium containing bFGF may be used as the bFGF-containing solution. A concentration of bFGF in the bFGF-containing solution is, for example, 1 ng/mL to 10000 ng/mL, preferably 10 ng/mL to 1000 ng/mL, and more preferably 100 ng/mL to 1000 ng/mL.

It is preferable that the amount of the bFGF-containing solution used in the binding process is an amount that makes one molecule to 10 molecules of bFGF exist per 100 molecules of the specific polypeptide. As for a volume, it is preferable that the volume is equal to or larger than an amount that causes the specific polypeptide to be entirely soaked by the solution.

It is preferable that the manufacturing method described above further includes a removing process of removing the bFGF-containing solution that has been brought into contact with the specific polypeptide, from the viewpoint of suppressing incorporation of bFGF into the liquid medium used in the case of culturing the cells. This process includes, for example, suctioning the bFGF-containing solution using a glass instrument. After removing the bFGF-containing solution, the specific polypeptide/bFGF complex may be washed with a phosphate buffer solution.

The manufacturing method described above may include a drying process of drying the specific polypeptide/bFGF complex. By performing this process, the scaffold material which is in the dry state is obtained.

The drying process may be performed together with the removing process. That is, the removing process of sufficiently removing the bFGF-containing solution also serves as the drying process, and through this process, the scaffold material which is in the dry state is obtained. Alternatively, the drying process may be a process of performing a drying treatment such as leaving the solution to stand in a low humidity environment, blast drying, and freeze-drying. These drying treatments may be performed after performing the binding process, or may be performed after performing the removing process.

The manufacturing method described above may include an impregnation process of impregnating the specific polypeptide/bFGF complex with the liquid which does not substantially contain bFGF. By performing this process, the scaffold material that is in the wet state in which the liquid that does not substantially contain bFGF is retained is obtained. The impregnation process may be performed after the binding process, after the removing process, or after the drying process.

Examples of the liquid that does not substantially contain bFGF include a phosphate buffer solution, a tris buffer solution, and physiological saline; a basal medium for mammalian cells, which is a liquid medium not containing bFGF; a liquid medium obtained by removing bFGF from the basal medium for mammalian cells; and the like.

The impregnation process is not necessarily required for obtaining the scaffold material that is in the wet state in which the liquid that does not substantially contain bFGF is retained. For example, in a case where bFGF in the bFGF-containing solution which has been brought into contact with the specific polypeptide is bound to the specific polypeptide in the binding process, and the solution that has been brought into contact with the specific polypeptide becomes substantially free of bFGF, the scaffold material that is in the wet state in which the liquid that does not substantially contain bFGF is retained is obtained.

### Examples

Hereinafter, the embodiments of the invention will be described in detail using Examples; however, the embodiments of the invention are not limited to these Examples.

Hereinbelow, "M" used for a concentration of a substance represents a molar concentration, and 1 M = 1 mol/L. In addition, regarding a concentration of a substance in a medium, "%" indicates "v/v%", unless otherwise particularly specified.

Hereinbelow, "PBS" refers to a phosphate buffer solution (phosphate buffered saline), and "EDTA" refers to ethylenediaminetetraacetic acid.

In each of Examples below, recombinant bFGF (Wako Pure Chemical Industries, Ltd.) was used as bFGF.

### <Example 1: Preparation of Specific Polypeptide >

DNA that encodes an amino acid sequence represented by SEQ ID No: 16 was amplified by a conventional method in which Polymerase Chain Reaction (PCR) is used. Hereinafter, a polypeptide consisting of the amino acid sequence represented by SEQ ID No: 16 is referred to as RCP-1. The amino acid sequence of RCP-1 corresponds to the 1^{st} to 55^{th}, 270^{th} to 277^{th}, and 295^{th} and 373^{rd} amino acid residues in an amino acid sequence represented by SEQ ID No: 1, the cysteine residue which is the 274^{th} residue is substituted by a serine residue, and a methionine residue which is added in a case where a polypeptide is prepared by a recombination technology is added to the N-terminal (refer to Table 2; an SMB domain, an RGD motif, and a heparin-binding domain are underlined). That is, the amino acid sequence of RCP-1 is a sequence obtained by adding a methionine residue to the N-terminal of an amino acid sequence represented by SEQ ID No: 9. The GRAVY value of RCP-1 is -1.072.

**[Table 2]**

| SEQ ID No. | Amino acid sequence | Number of amino acid residues | Corresponding position in SEQ ID No: 1 |
|---|---|---|---|
| 16 | | 143 | 1-55, 270-277, 295-373, C274S |

| | | | |
|---|---|---|---|
| *"." indicates that there are no corresponding amino acid residues when the polypeptide is aligned with SEQ ID No: 1. | | | |

An expression vector of RCP-1 was constructed by inserting DNA described above into a vector pET-28b (+) that has been cut by a restriction enzyme Ncol (TAKARA BIO INC.) in advance using In-Fusion Advantage PCR Cloning Kit (Clontech Laboratories, Inc.; "In-Fusion" is a registered trademark). The sequence of the expression vector was confirmed by sequence analysis.

The RCP-1 expression vector thus prepared was introduced into an E. coli competent cell BL21(DE3)pLysS (Novagen) by a conventional method, which was then applied onto a kanamycin-containing LB plate and incubated at 37°C for 16 hours. After confirming the introduction of the vector by a colony direct PCR method, 1 mM isopropyl-β-thiogalactopyranoside (Wako Pure Chemical Industries, Ltd.) was added thereto, shake culturing was performed at 37°C for 5 hours, and the expression of the polypeptide was induced.

Bacterial cells were collected by centrifugation, and the bacterial cells were resuspended in a wash buffer (20 mM Tris, 150 mM NaCl, and pH 7.6). The bacterial cells were subjected to ultrasonic breaking, centrifugation was performed at 4°C and at 15000 rpm for 30 minutes, and an insoluble fraction was collected. The insoluble fraction was washed with a wash buffer containing 0.5% Triton X100 and then resuspended in a low urea buffer (20 mM Tris, 150 mM NaCl, 2 M urea, and pH 7.6), and an ultrasonic wave was applied thereto. After an insoluble fraction was collected by centrifugation, a high urea buffer (High Urea Buffer: 20 mM Tris, 150 mM NaCl, 8 M urea, and pH 7.6) was added thereto, and an ultrasonic wave was applied, thereby solubilizing the insoluble fraction.

The solution containing RCP-1 obtained by the method described above was purified using AKTA Explorer100 (GE Healthcare) and HiTrap Heparin HP 5 mL (GE Healthcare; "HiTrap" is a registered trademark). Stepwise elution was performed using a high urea buffer as a binding buffer and a high salt concentration adjusting buffer (20 mM Tris, 1 M NaCl, 8 M urea, and pH 7.6) as an elution buffer, so as to purify RCP-1.

The purified RCP-1 solution was dialyzed using a disposable dialysis cassette Slide-A-Lyzer (molecular weight cutoff 3.5K) (Pierce Biotechnology Inc.; "Slide-A-Lyzer" is a registered trademark). A dialysis buffer (PBS, 1.5 M NaCl, 0.5 M L-arginine, 1 mM EDTA, and pH 7.4) served as the basic buffer of the of the outer dialysis fluid, and urea was removed in stepwise dialysis. The absorbance (wavelength 280 nm) of the final dialysis product was measured using a spectrophotometer NanoDrop (registered trademark, Thermo Fisher Scientific Inc.), so as to determine the concentration of RCP-1.

The aforementioned final dialysis product was used in Examples below. RCP-1 used for sample preparation in Examples below refer to the aforementioned final dialysis product.

### <Example 2: Evaluation of Binding between RCP-1 and bFGF (1)>

Essential 8 medium (containing bFGF at a concentration of 100 ng/mL; Life Technologies Corporation) and RCP-1 were mixed such that the concentration of RCP-1 becomes the concentrations shown in Table 3, and the mixture was left to stand at 37°C for 24 hours. After 24 hours, the amount of bFGF in the entire mixture was determined using Human bFGF ELISA Kit (RayBiotech, Inc.). The results are shown in Table 3.

Experiments were conducted in the same manner as described above using heparin, sodium dextran sulfate, or commercially available recombinant vitronectin VTN-N (Life Technologies Corporation) instead of RCP-1. Heparin and sodium dextran sulfate are known to stabilize bFGF by binding to bFGF. The amino acid sequence of VTN-N corresponds to the 43^{rd} to 459^{th} amino acid residues of the amino acid sequence represented by SEQ ID No: 1.

**[Table 3]**

| Additive | Concentration of additive | Amount of bFGF after 24 hours |
|---|---|---|
| - | - | 10.2% |
| Heparin | 25 µg/ml | 100.2% |
| Sodium dextran sulfate | 25 µg/ml | 100.5% |
| VTN-N | 3000 ng/ml | 6.7% |
| | 300 ng/ml | 8.9% |
| | 30 ng/ml | 9.5% |
| | 3 ng/ml | 10.6% |
| RCP-1 | 3000 ng/ml | 42.6% |
| | 300 ng/ml | 30.3% |
| | 30 ng/ml | 27.4% |
| | 3 ng/ml | 29.0% |

In a case where no additives were added to Essential 8 medium at all, approximately 90% of bFGF changed into a form that could not be detected by the ELISA method after 24 hours (bFGF is considered to be degraded, denatured, or inactivated). By the addition of heparin or sodium dextran sulfate, such changes in bFGF were almost completely suppressed.

In a case where VTN-N was added to Essential 8 medium, the amount of bFGF after 24 hours was approximately the same as or less than the amount thereof in a case where no additives were added. This result indicates that VTN-N cannot bind bFGF and cannot stabilize bFGF.

In a case where RCP-1 was added to Essential 8 medium, the amount of bFGF after 24 hours was approximately 30% to 40% of the initial amount, and as the concentration RCP-1 increased, the amount of bFGF after 24 hours was large. This result indicates that RCP-1 binds bFGF and stabilizes bFGF.

### <Example 3: Evaluation of Binding between RCP-1 and bFGF (2)>

An RCP-1 solution in which the RCP-1 concentration was 25 µg/mL was prepared by adding RCP-1 to a dialysis buffer. A bFGF aqueous solution in which the bFGF concentration was 100 ng/mL was separately prepared by adding bFGF to an injection solvent, and a bFGF aqueous solution obtained by a two-fold serial dilution with the injection solvent, a 10-fold diluted bFGF aqueous solution, and a 100-fold diluted bFGF aqueous solution were also prepared.

RCP-1 was adhered to the inner surface of each well of a polystyrene Falcon 96 well plate (Corning Incorporated; "Falcon" is a registered trademark) that has been subjected to a plasma treatment by adding 64 µL of the RCP-1 solution to each of the wells and leaving the solution to stand at 37°C for 2 hours. A supernatant was removed from each well by suction after leaving the solution to stand for 2 hours, 200 µL of the bFGF aqueous solution was added to each well, and the solution was left to stand at 37°C for 24 hours.

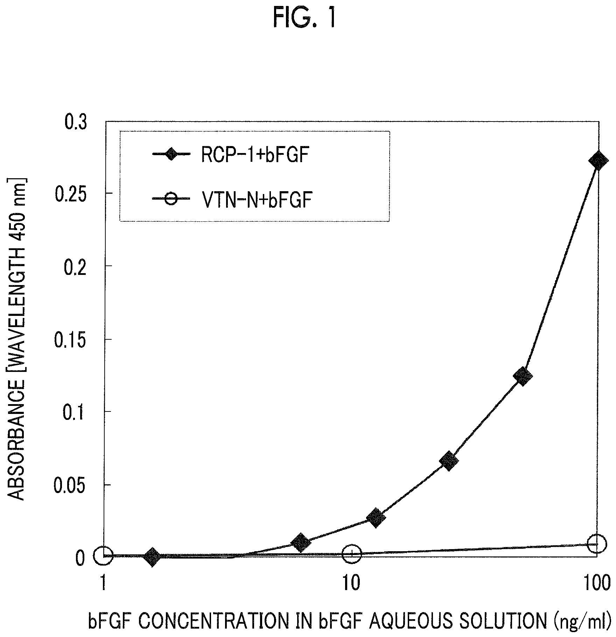

After leaving the solution to stand for 24 hours, a supernatant was removed from each well by suction, 300 µL of IMMUNOBLOCK (blocking agent, DS Pharma Biomedical Co., Ltd.) diluted to 20% using deionized water was added to each well, and the solution was left to stand at 25°C for 30 minutes. Thereafter, a supernatant was removed from each well by suction and washed three times with PBS-T (PBS with 0.5% Tween-20), and the absorbance (wavelength 450 nm) was measured using Human bFGF ELISA Kit (RayBiotech, Inc.). The results are showing in Fig. 1.

Experiments were conducted in the same manner as described above using recombinant vitronectin VTN-N (Life Technologies Corporation) instead of RCP-1.

As shown in Fig. 1, in a case where the bFGF aqueous solution was brought into contact with VTN-N in each well, an increase in the absorbance was not confirmed. On the other hand, in a case where the bFGF aqueous solution was brought into contact with RCP-1 in each well, the absorbance increased with the concentration of the bFGF aqueous solution. This result indicates that bFGF binds to RCP-1 adhered to the inner surface of each well.

### <Example 4: Evaluation of Effect of bFGF-Bound RCP-1 on iPS Cells (1)>

Human iPS cells 201B7 provided by iPS PORTAL, Inc. (Kyoto Mikuruma, 448-5 Kajii-cho, Imadegawa-Sagaru, Kawaramachi-dori, Kamigyo-ku, Kyoto, Japan) were used as pluripotent stem cells.

Maintenance culture of the human iPS cells was performed using TeSR-E8 (STEMCELL Technologies Inc.; "TeSR" is a registered trademark) as a liquid medium and a culture vessel coated with recombinant vitronectin VTN-N (Life Technologies Corporation) in an incubator at temperature 37°C/CO₂ concentration 5% (v/v; the same applies hereinafter). The liquid medium was replaced every day except for the day after seeding of the iPS cells. A passage operation was performed by treating the cells with a trypsin replacement TrypLE Select (Invitrogen Corporation) at 37°C for 5 minutes and dissociating the cells into single cells. Y-27362 ((R)-(+)-trans-N-(4-pyridyl)-4-(1-aminoethyl)-cyclohexanecarboxamide.2HCl·H₂O, Rho-associated protein kinase inhibitor, Wako Pure Chemical Industries, Ltd.) having a final concentration of 10 µM was added to the liquid medium in the case of dissociating the cells into single cells.

A cell suspension was prepared by dissociating the human iPS cells that have been subjected to maintenance culture into single cells and suspending the cells in Essential 8 medium (containing bFGF at a concentration of 100 ng/mL) or Essential 7 medium (having the same composition as Essential 8, except for being free of bFGF).

An RCP-1 solution in which the RCP-1 concentration was 25 µg/mL was prepared by adding RCP-1 to a dialysis buffer. RCP-1 was adhered to the inner surface of each well of a polystyrene Falcon 6 well plate (Corning Incorporated; "Falcon" is a registered trademark) that has been subjected to a plasma treatment by adding 1 mL of the RCP-1 solution to each of the wells and leaving the solution to stand at 37°C for 2 hours.

Wells having RCP-1 on the inner surfaces thereof were obtained by performing the above treatment. In addition, supernatants were removed from some of the wells by suction, 2 mL of Essential 8 medium was added thereto, the mixture was left to stand at 37°C for 24 hours, and bFGF was allowed to bind to RCP-1 on the inner surface of each well. Hereinafter, the well having RCP-1 is referred to as an "RCP-1 well", and a well treated with bFGF is referred to as a "bFGF-treated RCP-1 well".

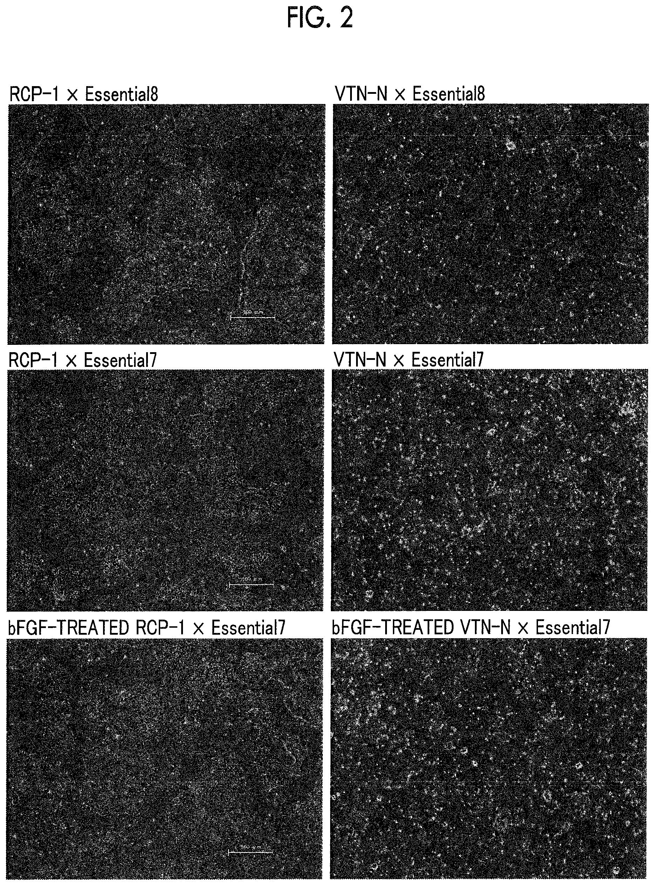

Supernatants were removed from the RCP-1 well and the bFGF-treated RCP-1 well by suction, and the cell suspension described above was seeded in each well such that the number of cells became 50000 cells/well. Subsequently, the human iPS cells were cultured while replacing the liquid medium (Essential 8 medium or Essential 7 medium) every day except for the day after the seeding. The numbers of cells five days after the seeding are shown in Table 4. In addition, the optical microscope images of the cells five days after the seeding are shown in Fig. 2.

Experiments were conducted in the same manner as described above using recombinant vitronectin VTN-N (Life Technologies Corporation) instead of RCP-1. Hereinafter, a well having VTN-N is referred to as a "VTN-N well", and a well treated with bFGF is referred to as a "bFGF-treated VTN-N well".

**[Table 4]**

| Culture vessel | Liquid medium for culture (concentration of bFGF) | Number of cells five days after seeding | Note |
|---|---|---|---|
| RCP-1 well | Essential 8 (100 ng/ml) | 2.4×10⁵ | Comparative Example |
| | Essential 7 (0 ng/ml) | 8.5×10⁴ | Comparative Example |
| bFGF-treated RCP-1 well | Essential 7 (0 n/ml) | 2.6×10⁵ | Present embodiment |
| VTN-N well | Essential 8 (100 ng/ml) | 2.5×10⁵ | Comparative Example |
| | Essential 7 (0 ng/ml) | 7.2×10⁴ | Comparative Example |
| bFGF-treated VTN-N well | Essential 7 (0 ng/ml) | 5.7×10⁴ | Comparative Example |

As shown in Table 4, in a case where the human iPS cells were cultured in a bFGF-containing liquid medium (Essential 8 medium), the cells stably proliferated both in the case of using the RCP-1 well and in the case of using the VTN-N well, and the numbers of cells were 2.4×10⁵ and 2.5×10⁵, respectively. On the other hand, in a case where the human iPS cells were cultured in a bFGF-free liquid medium (Essential 7 medium), cell proliferation was suppressed both in the case of using the RCP-1 well and in the case of using the VTN-N well, and the numbers of cells were 8.5×10⁴ (approximately 35%) and 7.2×10⁴ (approximately 29%), respectively.

In a case where the human iPS cells were cultured in the bFGF-free liquid medium (Essential 7 medium), the cell proliferation was further suppressed in the case of using the bFGF-treated VTN-N well, and the number of cells was 5.7×10⁴ (approximately 23%). On the other hand, even in a case where the human iPS cells were cultured in the bFGF-free liquid medium (Essential 7 medium), the cells stably proliferated in the case of using the bFGF-treated RCP-1 well, and the number of cells was 2.6×10⁵ (approximately 108%).

The results described above indicate that RCP-1 adhered to the inner surface of the culture vessel stabilizes bFGF by binding bFGF, and proliferation of iPS cells is promoted by a signal from bFGF bound to RCP-1.

As can be understood from Fig. 2, the human iPS cells had the form that was characteristic to human iPS cells (occupancy percentage of a nucleus was high, and the cell boundary was unclear), and the undifferentiated state thereof was maintained, in a case where the cells were cultured in the bFGF-free liquid medium (Essential 7 medium) using the bFGF-treated RCP-1 well.

### <Example 5: Evaluation of Effect of bFGF-Bound RCP-1 on iPS Cells (2)>

A liquid medium in which the bFGF concentration was 1000 ng/mL or 100 ng/mL was prepared by adding bFGF to Essential 7 medium.

Using the same method as in Example 4, a well having RCP-1 on the inner surface thereof (RCP-1 well) was prepared, 2 mL of the liquid medium was added to each well, and the medium was left to stand at 37°C for 24 hours, and bFGF was allowed to bind to RCP-1 on the inner surface of each well. Supernatants were removed by suction, and then a cell suspension obtained by suspending human iPS cells in Essential 7 medium was seeded in each well such that the number of cells became 50000 cells/well. Subsequently, the human iPS cells were cultured while replacing the liquid medium (Essential 7 medium) every day except for the day after the seeding. The numbers of cells five days after the seeding are shown in Table 5.

**[Table 5]**

| Concentration of bFGF in liquid medium used for treatment of RCP-1 well | Liquid medium for culture (concentration of bFGF) | Number of cells five days after seeding | Note |
|---|---|---|---|
| 1000 ng/ml | Essential 7 (0 ng/ml) | 2.6×10⁵ | Present embodiment |
| 100 ng/ml | Essential 7 (0 ng/ml) | 2.4×10⁵ | Present embodiment |

As shown in Table 5, the human iPS cells stably proliferated by using the well having RCP-1 to which bFGF is bound, even in a case where the liquid medium did not contain bFGF.

### <Example 6: Evaluation of Effect of bFGF-Bound RCP-1 on iPS Cells (3)>

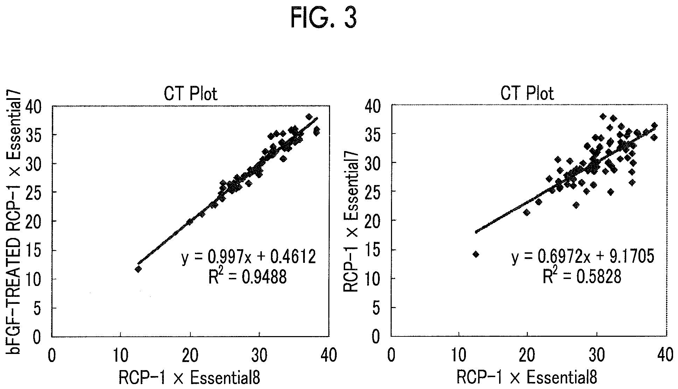

The human iPS cells cultured in Example 4 were detached and collected, and mRNA was purified using an RNA purification kit NucleoSpin RNA (TAKARA BIO INC.). mRNA thus obtained was subjected to reverse transcription using a reverse transcription reaction reagent PrimeScript RT Master Mix (TAKARA BIO INC.; "PrimeScript" is a registered trademark) so as to obtain cDNA, and comparative assessment of the expression levels of 90 kinds of genes was performed by a cycle comparative method (ΔΔC_{T} method) using a real time PCR system TaqMan Array Human Stem Cell Pluripotency Panel (Life Technologies Corporation; "TaqMan" is a registered trademark). The results are shown in Fig. 3.

As shown in Fig. 3, the human iPS cells cultured in a bFGF-free liquid medium (Essential 7 medium) using an RCP-1 well and the human iPS cells cultured in a bFGF-containing liquid medium (Essential 8 medium) using the RCP-1 well are different in gene expression pattern, and this result indicates that differentiation of the human iPS cells has progressed in the absence of bFGF.

On the other hand, the human iPS cells cultured in the bFGF-free liquid medium (Essential 7 medium) using a bFGF-treated RCP-1 well and the human iPS cells cultured in a bFGF-containing liquid medium (Essential 8 medium) using the RCP-1 well show the same gene expression pattern. This result shows that the bFGF-treated RCP-1 well has the ability to maintain the characteristics of the human iPS cells at the same level as the bFGF-containing liquid medium (Essential 8 medium).

### <Example 7: Evaluation of Amount of bFGF Contained in Liquid Medium during Culture Process>

Using the same method as in Example 4, a well having RCP-1 (RCP-1 well) and a well further treated with bFGF (bFGF-treated RCP-1 well) were prepared.

Human iPS cells that have been subjected to maintenance culturing were dissociated into single cells and suspended in Essential 8 medium or Essential 7 medium, so as to prepare a cell suspension.

The cell suspension was seeded in the RCP-1 well and the bFGF-treated RCP-1 well such that the number of cells became 50000 cells/well. Culture supernatants were collected one day after the seeding, two days after the seeding, and three days after the seeding, absorbance (wavelength 450 nm) of each of the culture supernatants was measured using Human bFGF ELISA Kit (RayBiotech, Inc.), and amount of bFGF in each of the culture supernatants was determined. The results are shown in Table 6.

**[Table 6]**

| Culture vessel | Liquid medium for culture (concentration of bFGF) | One day after seeding | Two days after seeding | Three days after seeding | Note |
|---|---|---|---|---|---|
| RCP-1 well | Essential 8 (100 n/ml) | 131.7 | 75.1 | 77.9 | Comparative Example |
| | Essential 7 (0 ng/ml) | ND | ND | ND | Comparative Example |
| bFGF-treated RCP-1 well | Essential 7 (0 ng/ml) | ND | ND | ND | Present embodiment |

As shown in Table 6, in a case where the human iPS cells were cultured in a bFGF-free liquid medium (Essential 7 medium) using the bFGF-treated RCP-1 well, bFGF was not detected in the culture supernatants. This result indicates that bFGF bound to RCP-1 has not transferred to the liquid medium in the cell culture.

## Claims

1. A method for culturing pluripotent stem cells comprising:
a culturing process, which is performed in a liquid medium which does not substantially contain a basic fibroblast growth factor: the process of bringing pluripotent stem cells into contact with a polypeptide which has cell adhesion activity and includes a first domain for binding the basic fibroblast growth factor and a second domain for adhering to a culture vessel and culturing the pluripotent stem cells,
wherein the basic fibroblast growth factor is bound to the first domain, and the second domain is adhered to the culture vessel,
wherein the number of amino acid residues of the polypeptide is 80 to 250,
wherein the first domain includes any one of the following sequence A-1, sequence A-2, and sequence A-3, and
the second domain includes any one of the following sequence B-1, sequence B-2, and sequence B-3.
Sequence A-1: an amino acid sequence represented by SEQ ID No: 2
Sequence A-2: an amino acid sequence which shares identity of equal to or higher than 80% with the amino acid sequence represented by SEQ ID No: 2 and has the ability to bind the basic fibroblast growth factor
Sequence A-3: an amino acid sequence which is obtained by addition, substitution, or deletion of one or several amino acid residues in the amino acid sequence represented by SEQ ID No: 2 and has the ability to bind the basic fibroblast growth factor
Sequence B-1: an amino acid sequence represented by SEQ ID No: 3
Sequence B-2: an amino acid sequence which shares identity of equal to or higher than 80% with the amino acid sequence represented by SEQ ID No: 3 and has the ability to adhere to the culture vessel
Sequence B-3: an amino acid sequence which is obtained by addition, substitution, or deletion of one or several amino acid residues in the amino acid sequence represented by SEQ ID No: 3 and has the ability to adhere to the culture vessel.

2. The method for culturing pluripotent stem cells according to claim 1, wherein the first domain has a negative charge.

3. The method for culturing pluripotent stem cells according to any one of claims 1 or 2, wherein the polypeptide further includes an RGD motif.

4. The method for culturing pluripotent stem cells according to any one of claims 1 to 3, further comprising, before the culturing process:
an adhering process of bringing the culture vessel into contact with the polypeptide which has cell adhesion activity and includes the first domain for binding the basic fibroblast growth factor and the second domain for adhering to the culture vessel and adhering the second domain to the culture vessel; and
a binding process of bringing the polypeptide into contact with a solution containing the basic fibroblast growth factor and allowing the basic fibroblast growth factor to be bound to the first domain,
preferably further comprising, before the culturing process:
a removing process of removing the solution which is brought into contact with the polypeptide.

5. A method for manufacturing a culture vessel by which a culture vessel including a polypeptide that has cell adhesion activity and has a basic fibroblast growth factor bound thereto is manufactured, the method comprising:
an adhering process of bringing the culture vessel into contact with the polypeptide which has cell adhesion activity and includes a first domain for binding the basic fibroblast growth factor and a second domain for adhering to the culture vessel and adhering the second domain to the culture vessel; and
a binding process of bringing the polypeptide into contact with a solution containing the basic fibroblast growth factor and allowing the basic fibroblast growth factor to be bound to the first domain,
wherein the number of amino acid residues of the polypeptide is 80 to 250,
wherein the first domain includes any one of the following sequence A-1, sequence A-2, and sequence A-3, and
the second domain includes any one of the following sequence B-1, sequence B-2, and sequence B-3.
Sequence A-1: an amino acid sequence represented by SEQ ID No: 2
Sequence A-2: an amino acid sequence which shares identity of equal to or higher than 80% with the amino acid sequence represented by SEQ ID No: 2 and has the ability to bind the basic fibroblast growth factor
Sequence A-3: an amino acid sequence which is obtained by addition, substitution, or deletion of one or several amino acid residues in the amino acid sequence represented by SEQ ID No: 2 and has the ability to bind the basic fibroblast growth factor
Sequence B-1: an amino acid sequence represented by SEQ ID No: 3
Sequence B-2: an amino acid sequence which shares identity of equal to or higher than 80% with the amino acid sequence represented by SEQ ID No: 3 and has the ability to adhere to the culture vessel
Sequence B-3: an amino acid sequence which is obtained by addition, substitution, or deletion of one or several amino acid residues in the amino acid sequence represented by SEQ ID No: 3 and has the ability to adhere to the culture vessel.

6. The method for manufacturing a culture vessel according to claim 5,
wherein a content of the basic fibroblast growth factor in the solution is in a range of 100 ng/mL to 1000 ng/mL.

7. The method for manufacturing a culture vessel according to any one of claims 5 or 6, further comprising:
a drying process of drying the polypeptide which is adhered to the culture vessel and has the basic fibroblast growth factor bound thereto; and/or
an impregnation process of impregnating the polypeptide which is adhered to the culture vessel and has the basic fibroblast growth factor bound thereto with a liquid which does not substantially contain the basic fibroblast growth factor; and/or
a removing process of removing the solution which is brought into contact with the polypeptide.

8. The method for manufacturing a culture vessel according to any one of claims 5 to 7, wherein the polypeptide further includes an RGD motif.

9. A culture vessel comprising:
a polypeptide which has cell adhesion activity and includes a first domain for binding a basic fibroblast growth factor and a second domain for adhering to a culture vessel,
wherein the basic fibroblast growth factor is bound to the first domain and the second domain is adhered to the culture vessel,
wherein the number of amino acid residues of the polypeptide is 80 to 250,
wherein the first domain includes any one of the following sequence A-1, sequence A-2, and sequence A-3, and
the second domain includes any one of the following sequence B-1, sequence B-2, and sequence B-3.
Sequence A-1: an amino acid sequence represented by SEQ ID No: 2
Sequence A-2: an amino acid sequence which shares identity of equal to or higher than 80% with the amino acid sequence represented by SEQ ID No: 2 and has the ability to bind the basic fibroblast growth factor
Sequence A-3: an amino acid sequence which is obtained by addition, substitution, or deletion of one or several amino acid residues in the amino acid sequence represented by SEQ ID No: 2 and has the ability to bind the basic fibroblast growth factor
Sequence B-1: an amino acid sequence represented by SEQ ID No: 3
Sequence B-2: an amino acid sequence which shares identity of equal to or higher than 80% with the amino acid sequence represented by SEQ ID No: 3 and has the ability to adhere to the culture vessel
Sequence B-3: an amino acid sequence which is obtained by addition, substitution, or deletion of one or several amino acid residues in the amino acid sequence represented by SEQ ID No: 3 and has the ability to adhere to the culture vessel.

10. The culture vessel according to claim 9,
wherein the polypeptide is in a dry state, or
wherein the polypeptide is in a wet state in which a liquid that does not substantially contain the basic fibroblast growth factor is retained.

11. The culture vessel according to any one of claims 9 or 10,
wherein the polypeptide further includes an RGD motif.

12. A scaffold material for culturing cells, comprising:
a polypeptide which has cell adhesion activity and includes a first domain for binding a basic fibroblast growth factor and a second domain for adhering to a culture vessel,
wherein the basic fibroblast growth factor is bound to the first domain,
wherein the number of amino acid residues of the polypeptide is 80 to 250,
wherein the first domain includes any one of the following sequence A-1, sequence A-2, and sequence A-3, and
the second domain includes any one of the following sequence B-1, sequence B-2, and sequence B-3.
Sequence A-1: an amino acid sequence represented by SEQ ID No: 2
Sequence A-2: an amino acid sequence which shares identity of equal to or higher than 80% with the amino acid sequence represented by SEQ ID No: 2 and has the ability to bind the basic fibroblast growth factor
Sequence A-3: an amino acid sequence which is obtained by addition, substitution, or deletion of one or several amino acid residues in the amino acid sequence represented by SEQ ID No: 2 and has the ability to bind the basic fibroblast growth factor
Sequence B-1: an amino acid sequence represented by SEQ ID No: 3
Sequence B-2: an amino acid sequence which shares identity of equal to or higher than 80% with the amino acid sequence represented by SEQ ID No: 3 and has the ability to adhere to the culture vessel
Sequence B-3: an amino acid sequence which is obtained by addition, substitution, or deletion of one or several amino acid residues in the amino acid sequence represented by SEQ ID No: 3 and has the ability to adhere to the culture vessel.

13. The scaffold material for culturing cells according to claim 12,
which is in a dry state, or
which is in a wet state in which a liquid that does not substantially contain the basic fibroblast growth factor is retained.

14. The scaffold material for culturing cells according to any one of claims 12 to 13,
wherein the polypeptide further includes an RGD motif.

## Patentansprüche

1. Verfahren zum Kultivieren pluripotenter Stammzellen, umfassend:
einen Kultivierungsprozess, welcher in einem Flüssigmedium durchgeführt wird, welches im Wesentlichen keinen basischen Fibroblasten-Wachstumsfaktor (basic fibroblast growth factor) enthält: der Prozess des Inkontaktbringens von pluripotenten Stammzellen mit einem Polypeptid, welches Zelladhäsionsaktivität hat und
eine erste Domäne einschließt, um den basischen Fibroblasten-Wachstumsfaktor zu binden, und eine zweite Domäne einschließt, um an ein Kulturgefäß zu adhärieren, und des Kultivierens der pluripotenten Stammzellen,
wobei der basische Fibroblasten-Wachstumsfaktor an die erste Domäne gebunden ist, und die zweite Domäne an das Kulturgefäß adhäriert ist,
wobei die Anzahl der Aminosäurereste des Polypeptids 80 bis 250 beträgt,
wobei die erste Domäne irgendeine der folgenden einschließt: Sequenz A-1, Sequenz A-2 und Sequenz A-3, und
die zweite Domäne irgendeine der folgenden einschließt:
Sequenz B-1, Sequenz B-2 und Sequenz B-3.
Sequenz A-1: eine durch SEQ ID No: 2 repräsentierte Aminosäuresequenz
Sequenz A-2: eine Aminosäuresequenz, welche eine Identität von gleich oder höher als 80% mit der durch SEQ ID No: 2 repräsentierten Aminosäuresequenz aufweist, und die Fähigkeit hat, den basischen Fibroblasten-Wachstumsfaktor zu binden
Sequenz A-3: eine Aminosäuresequenz, welche durch Addition, Substitution oder Deletion eines oder mehrerer Aminosäurereste(s) in der durch SEQ ID No: 2 repräsentierten Aminosäuresequenz erhalten wird, und die Fähigkeit hat, den basischen Fibroblasten-Wachstumsfaktor zu binden
Sequenz B-1: eine durch SEQ ID No: 3 repräsentierte Aminosäuresequenz
Sequenz B-2: eine Aminosäuresequenz, welche eine Identität von gleich oder höher als 80% mit der durch SEQ ID No: 3 repräsentierten Aminosäuresequenz aufweist, und die Fähigkeit hat, an das Kulturgefäß zu adhärieren Sequenz B-3: eine Aminosäuresequenz, welche durch Addition, Substitution oder Deletion eines oder mehrerer Aminosäurereste(s) in der durch SEQ ID No: 3 repräsentierten Aminosäuresequenz erhalten wird, und die Fähigkeit hat, an das Kulturgefäß zu adhärieren.

2. Verfahren zum Kultivieren pluripotenter Stammzellen gemäß Anspruch 1, wobei die erste Domäne eine negative Ladung aufweist.

3. Verfahren zum Kultivieren pluripotenter Stammzellen gemäß irgendeinem der Ansprüche 1 oder 2,
wobei das Polypeptid weitergehend ein RGD-Motiv einschließt.

4. Verfahren zum Kultivieren pluripotenter Stammzellen gemäß irgendeinem der Ansprüche 1 bis 3, weitergehend umfassend, vor dem Kultivierungsprozess:
einen Adhärierungsprozess des Inkontaktbringens des Kulturgefäßes mit dem Polypeptid, welches Zelladhäsionsaktivität hat und die erste Domäne einschließt, um den basischen Fibroblasten-Wachstumsfaktor zu binden, und die zweite Domäne einschließt, um an das Kulturgefäß zu adhärieren, und des Adhärierens der zweiten Domäne an das Kulturgefäß; und
einen Bindungsprozess des Inkontaktbringens des Polypeptids mit einer Lösung, welche den basischen Fibroblasten-Wachstumsfaktor enthält, und des Ermöglichens des basischen Fibroblasten-Wachstumsfaktors, an die erste Domäne gebunden zu sein, bevorzugt weitergehend umfassend, vor dem Kultivierungsprozess:
einen Entfernungsprozess des Entfernens der Lösung, welche mit dem Polypeptid in Kontakt gebracht wird.

5. Verfahren zum Herstellen eines Kulturgefäßes, durch welches ein Kulturgefäß hergestellt wird, welches ein Polypeptid einschließt, welches Zelladhäsionsaktivität hat und einen daran gebundenen basischen Fibroblasten-Wachstumsfaktor aufweist, das Verfahren umfassend:
einen Adhärierungsprozess des Inkontaktbringens des Kulturgefäßes mit dem Polypeptid, welches Zelladhäsionsaktivität hat und eine erste Domäne einschließt, um den basischen Fibroblasten-Wachstumsfaktor zu binden, und eine zweite Domäne einschließt, um an das Kulturgefäß zu adhärieren, und des Adhärierens der zweiten Domäne an das Kulturgefäß; und
einen Bindungsprozess des Inkontaktbringens des Polypeptids mit einer Lösung, welche den basischen Fibroblasten-Wachstumsfaktor enthält, und des Ermöglichens des basischen Fibroblasten-Wachstumsfaktors, an die erste Domäne gebunden zu sein,
wobei die Anzahl der Aminosäurereste des Polypeptids 80 bis 250 beträgt,
wobei die erste Domäne irgendeine der folgenden einschließt: Sequenz A-1, Sequenz A-2 und Sequenz A-3, und
die zweite Domäne irgendeine der folgenden einschließt:
Sequenz B-1, Sequenz B-2 und Sequenz B-3.
Sequenz A-1: eine durch SEQ ID No: 2 repräsentierte Aminosäuresequenz
Sequenz A-2: eine Aminosäuresequenz, welche eine Identität von gleich oder höher als 80% mit der durch SEQ ID No: 2 repräsentierten Aminosäuresequenz aufweist, und die Fähigkeit hat, den basischen Fibroblasten-Wachstumsfaktor zu binden
Sequenz A-3: eine Aminosäuresequenz, welche durch Addition, Substitution oder Deletion eines oder mehrerer Aminosäurereste(s) in der durch SEQ ID No: 2 repräsentierten Aminosäuresequenz erhalten wird, und die Fähigkeit hat, den basischen Fibroblasten-Wachstumsfaktor zu binden
Sequenz B-1: eine durch SEQ ID No: 3 repräsentierte Aminosäuresequenz
Sequenz B-2: eine Aminosäuresequenz, welche eine Identität von gleich oder höher als 80% mit der durch SEQ ID No: 3 repräsentierten Aminosäuresequenz aufweist, und die Fähigkeit hat, an das Kulturgefäß zu adhärieren Sequenz B-3: eine Aminosäuresequenz, welche durch Addition, Substitution oder Deletion eines oder mehrerer Aminosäurereste(s) in der durch SEQ ID No: 3 repräsentierten Aminosäuresequenz erhalten wird, und die Fähigkeit hat, an das Kulturgefäß zu adhärieren.

6. Verfahren zum Herstellen eines Kulturgefäßes gemäß Anspruch 5,
wobei ein Gehalt des basischen Fibroblasten-Wachstumsfaktors in der Lösung in einem Bereich von 100 ng/mL bis 1000 ng/mL liegt.

7. Verfahren zum Herstellen eines Kulturgefäßes gemäß irgendeinem der Ansprüche 5 oder 6, weitergehend umfassend:
einen Trocknungsprozess des Trocknens des Polypeptids, welches an das Kulturgefäß adhäriert ist, und an welches der basische Fibroblasten-Wachstumsfaktor gebunden ist; und/oder
einen Imprägnierungsprozess des Imprägnierens des Polypeptids, welches an das Kulturgefäß adhäriert ist und an welches der basische Fibroblasten-Wachstumsfaktor gebunden ist, mit einer Flüssigkeit, welche im Wesentlichen den basischen Fibroblasten-Wachstumsfaktor nicht enthält; und/oder
einen Entfernungsprozess des Entfernens der Lösung, welche mit dem Polypeptid in Kontakt gebracht wird.

8. Verfahren zum Herstellen eines Kulturgefäßes gemäß irgendeinem der Ansprüche 5 bis 7,
wobei das Polypeptid weitergehend ein RGD-Motiv einschließt.

9. Kulturgefäß, umfassend:
ein Polypeptid, welches Zelladhäsionsaktivität hat und eine erste Domäne einschließt, um einen basischen Fibroblasten-Wachstumsfaktor zu binden, und eine zweite Domäne einschließt, um an ein Kulturgefäß zu adhärieren, wobei der basische Fibroblasten-Wachstumsfaktor an die erste Domäne gebunden ist und die zweite Domäne an das Kulturgefäß adhäriert ist,
wobei die Anzahl der Aminosäurereste des Polypeptids 80 bis 250 beträgt,
wobei die erste Domäne irgendeine der folgenden einschließt: Sequenz A-1, Sequenz A-2 und Sequenz A-3, und
die zweite Domäne irgendeine der folgenden einschließt:
Sequenz B-1, Sequenz B-2 und Sequenz B-3.
Sequenz A-1: eine durch SEQ ID No: 2 repräsentierte Aminosäuresequenz
Sequenz A-2: eine Aminosäuresequenz, welche eine Identität von gleich oder höher als 80% mit der durch SEQ ID No: 2 repräsentierten Aminosäuresequenz aufweist, und die Fähigkeit hat, den basischen Fibroblasten-Wachstumsfaktor zu binden
Sequenz A-3: eine Aminosäuresequenz, welche durch Addition, Substitution oder Deletion eines oder mehrerer Aminosäurereste(s) in der durch SEQ ID No: 2 repräsentierten Aminosäuresequenz erhalten wird, und die Fähigkeit hat, den basischen Fibroblasten-Wachstumsfaktor zu binden
Sequenz B-1: eine durch SEQ ID No: 3 repräsentierte Aminosäuresequenz
Sequenz B-2: eine Aminosäuresequenz, welche eine Identität von gleich oder höher als 80% mit der durch SEQ ID No: 3 repräsentierten Aminosäuresequenz aufweist, und die Fähigkeit hat, an das Kulturgefäß zu adhärieren
Sequenz B-3: eine Aminosäuresequenz, welche durch Addition, Substitution oder Deletion eines oder mehrerer Aminosäurereste(s) in der durch SEQ ID No: 3 repräsentierten Aminosäuresequenz erhalten wird, und die Fähigkeit hat, an das Kulturgefäß zu adhärieren.

10. Kulturgefäß gemäß Anspruch 9,
wobei das Polypeptid in einem trockenen Zustand vorliegt, oder
wobei das Polypeptid in einem nassen Zustand vorliegt, in welchem eine Flüssigkeit gebunden ist, welche im Wesentlichen den basischen Fibroblasten-Wachstumsfaktor nicht enthält.

11. Kulturgefäß gemäß irgendeinem der Ansprüche 9 oder 10, wobei das Polypeptid weitergehend ein RGD-Motiv einschließt.

12. Trägermaterial (scaffold material) zum Kultivieren von Zellen, umfassend:
ein Polypeptid, welches Zelladhäsionsaktivität hat und eine erste Domäne einschließt, um einen basischen Fibroblasten-Wachstumsfaktor zu binden, und eine zweite Domäne einschließt, um an ein Kulturgefäß zu adhärieren,
wobei der basische Fibroblasten-Wachstumsfaktor an die erste Domäne gebunden ist,
wobei die Anzahl der Aminosäurereste des Polypeptids 80 bis 250 beträgt,
wobei die erste Domäne irgendeine der folgenden einschließt: Sequenz A-1, Sequenz A-2 und Sequenz A-3, und
die zweite Domäne irgendeine der folgenden einschließt:
Sequenz B-1, Sequenz B-2 und Sequenz B-3.
Sequenz A-1: eine durch SEQ ID No: 2 repräsentierte Aminosäuresequenz
Sequenz A-2: eine Aminosäuresequenz, welche eine Identität von gleich oder höher als 80% mit der durch SEQ ID No: 2 repräsentierten Aminosäuresequenz aufweist, und die Fähigkeit hat, den basischen Fibroblasten-Wachstumsfaktor zu binden
Sequenz A-3: eine Aminosäuresequenz, welche durch Addition, Substitution oder Deletion eines oder mehrerer Aminosäurereste(s) in der durch SEQ ID No: 2 repräsentierten Aminosäuresequenz erhalten wird, und die Fähigkeit hat, den basischen Fibroblasten-Wachstumsfaktor zu binden
Sequenz B-1: eine durch SEQ ID No: 3 repräsentierte Aminosäuresequenz
Sequenz B-2: eine Aminosäuresequenz, welche eine Identität von gleich oder höher als 80% mit der durch SEQ ID No: 3 repräsentierten Aminosäuresequenz aufweist, und die Fähigkeit hat, an das Kulturgefäß zu adhärieren
Sequenz B-3: eine Aminosäuresequenz, welche durch Addition, Substitution oder Deletion eines oder mehrerer Aminosäurereste(s) in der durch SEQ ID No: 3 repräsentierten Aminosäuresequenz erhalten wird, und die Fähigkeit hat, an das Kulturgefäß zu adhärieren.

13. Trägermaterial zum Kultivieren von Zellen gemäß Anspruch 12,
welches in einem trockenen Zustand vorliegt, oder welches in einem nassen Zustand vorliegt, in welchem eine Flüssigkeit gebunden ist, welche im Wesentlichen den basischen Fibroblasten-Wachstumsfaktor nicht enthält.

14. Trägermaterial zum Kultivieren von Zellen gemäß irgendeinem der Ansprüche 12 bis 13,
wobei das Polypeptid weitergehend ein RGD-Motiv einschließt.

## Revendications

1. Procédé de mise en culture de cellules souches pluripotentes comprenant :
un processus de mise en culture, qui est réalisé dans un milieu liquide qui ne contient sensiblement pas de facteur de croissance fibroblastique basique : le processus de mise en contact de cellules souches pluripotentes avec un polypeptide qui présente une activité d'adhérence cellulaire et inclut un premier domaine destiné à lier le facteur de croissance fibroblastique basique à un second domaine pour se fixer à un récipient de culture et mettre en culture les cellules souches pluripotentes,
dans lequel le facteur de croissance fibroblastique basique est lié au premier domaine, et le second domaine est fixé au récipient de culture,
dans lequel le nombre de résidus d'acides aminés du polypeptide va de 80 à 250,
dans lequel le premier domaine inclut l'une quelconque de la séquence A-1, la séquence A-2 et la séquence A-3 suivantes, et
le second domaine inclut l'une quelconque de la séquence B-1, la séquence B-2 et la séquence B-3 suivantes ;
Séquence A-1 : une séquence d'acides aminés représentée par la SÉQ ID N° 2
Séquence A-2: une séquence d'acides aminés qui partage l'identité d'égale ou supérieure à 80 % avec la séquence d'acides aminés représentée par la SÉQ ID N° 2 et est capable de se lier au facteur de croissance fibroblastique basique
Séquence A-3 : une séquence d'acides aminés qui est obtenue par addition, substitution ou délétion d'un ou plusieurs résidus d'acides aminés dans la séquence d'acides aminés représentée par la SÉQ ID N° 2 et est capable de se lier au facteur de croissance fibroblastique basique
Séquence B-1 : une séquence d'acides aminés représentée par la SÉQ ID N° 3
Séquence B-2: une séquence d'acides aminés qui partage l'identité d'égale ou supérieure à 80 % avec la séquence d'acides aminés représentée par la SÉQ ID N° 3 et est capable de se fixer au récipient de culture
Séquence B-3: une séquence d'acides aminés qui est obtenue par addition, substitution ou délétion d'un ou plusieurs résidus d'acides aminés dans la séquence d'acides aminés représentée par la SÉQ ID N° 3 et est capable de se fixer au récipient de culture.

2. Procédé de mise en culture de cellules souches pluripotentes selon la revendication 1, dans lequel le premier domaine présente une charge négative.

3. Procédé de mise en culture de cellules souches pluripotentes selon l'une quelconque des revendications 1 ou 2, dans lequel le polypeptide inclut en outre un motif RGD.

4. Procédé de mise en culture de cellules souches pluripotentes selon l'une quelconque des revendications 1 à 3, comprenant en outre, avant le processus de mise en culture :
un processus de fixation consistant à mettre en contact le récipient de culture avec le polypeptide qui présente une activité d'adhérence cellulaire et inclut le premier domaine pour lier le facteur de croissance fibroblastique basique au second domaine pour se fixer au récipient de culture et fixer le second domaine au récipient de culture ; et
un processus de liaison consistant à mettre en contact le polypeptide avec une solution contenant le facteur de croissance fibroblastique basique et permettre au facteur de croissance fibroblastique basique d'être lié au premier domaine,
de préférence comprenant en outre, avant le processus de mise en culture :
un processus de retrait consistant à retirer la solution qui est mise en contact avec le polypeptide.

5. Procédé de fabrication d'un récipient de culture par lequel un récipient de culture incluant un polypeptide qui présente une activité d'adhérence cellulaire et présente un facteur de croissance fibroblastique basique qui y est lié est fabriqué, le procédé comprenant :
un processus de fixation consistant à mettre en contact le récipient de culture avec le polypeptide qui présente une activité d'adhérence cellulaire et inclut un premier domaine destiné à lier le facteur de croissance fibroblastique basique à un second domaine pour se fixer au récipient de culture et fixer le second domaine au récipient de culture ; et
un processus de liaison consistant à mettre en contact le polypeptide avec une solution contenant le facteur de croissance fibroblastique basique et permettre au facteur de croissance fibroblastique basique d'être lié au premier domaine,
dans lequel le nombre de résidus d'acides aminés du polypeptide va de 80 à 250,
dans lequel le premier domaine inclut l'une quelconque de la séquence A-1, la séquence A-2 et la séquence A-3 suivantes, et
le second domaine inclut l'une quelconque de la séquence B-1, la séquence B-2 et la séquence B-3 suivantes ;
Séquence A-1 : une séquence d'acides aminés représentée par la SÉQ ID N° 2
Séquence A-2 : une séquence d'acides aminés qui partage l'identité d'égale ou supérieure à 80 % avec la séquence d'acides aminés représentée par la SÉQ ID N° 2 et est capable de se lier au facteur de croissance fibroblastique basique
Séquence A-3 : une séquence d'acides aminés qui est obtenue par addition, substitution ou délétion d'un ou plusieurs résidus d'acides aminés dans la séquence d'acides aminés représentée par la SÉQ ID N° 2 et est capable de se lier au facteur de croissance fibroblastique basique
Séquence B-1 : une séquence d'acides aminés représentée par la SÉQ ID N° 3
Séquence B-2 : une séquence d'acides aminés qui partage l'identité d'égale ou supérieure à 80 % avec la séquence d'acides aminés représentée par la SÉQ ID N° 3 et est capable de se fixer au récipient de culture
Séquence B-3 : une séquence d'acides aminés qui est obtenue par addition, substitution ou délétion d'un ou plusieurs résidus d'acides aminés dans la séquence d'acides aminés représentée par la SÉQ ID N° 3 et est capable de se fixer au récipient de culture.

6. Procédé de fabrication d'un récipient de culture selon la revendication 5,
dans lequel une teneur du facteur de croissance fibroblastique basique dans la solution se situe dans une plage allant de 100 ng/ml à 1 000 ng/ml.

7. Procédé de fabrication d'un récipient de culture selon l'une quelconque des revendications 5 ou 6, comprenant en outre :
un processus de séchage consistant à sécher le polypeptide qui est fixé au récipient de culture et a le facteur de croissance fibroblastique basique lié à celui-ci ; et/ou
un processus d'imprégnation consistant à imprégner le polypeptide qui est fixé au récipient de culture et a le facteur de croissance fibroblastique basique lié à celui-ci avec un liquide qui ne contient sensiblement pas le facteur de croissance fibroblastique basique ; et/ou
un processus de retrait consistant à retirer la solution qui est mise en contact avec le polypeptide.

8. Procédé de fabrication d'un récipient de culture selon l'une quelconque des revendications 5 à 7, dans lequel le polypeptide inclut en outre un motif RGD.

9. Récipient de culture comprenant :
un polypeptide qui présente une activité d'adhérence cellulaire et inclut un premier domaine destiné à lier un facteur de croissance fibroblastique basique à un second domaine pour se fixer à un récipient de culture,
dans lequel le facteur de croissance fibroblastique basique est lié au premier domaine et le second domaine est fixé au récipient de culture,
dans lequel le nombre de résidus d'acides aminés du polypeptide va de 80 à 250,
dans lequel le premier domaine inclut l'une quelconque de la séquence A-1, la séquence A-2 et la séquence A-3 suivantes, et
le second domaine inclut l'une quelconque de la séquence B-1, la séquence B-2 et la séquence B-3 suivantes ;
Séquence A-1 : une séquence d'acides aminés représentée par la SÉQ ID N° 2
Séquence A-2 : une séquence d'acides aminés qui partage l'identité d'égale ou supérieure à 80 % avec la séquence d'acides aminés représentée par la SÉQ ID N° 2 et est capable de se lier au facteur de croissance fibroblastique basique
Séquence A-3 : une séquence d'acides aminés qui est obtenue par addition, substitution ou délétion d'un ou plusieurs résidus d'acides aminés dans la séquence d'acides aminés représentée par la SÉQ ID N° 2 et est capable de se lier au facteur de croissance fibroblastique basique
Séquence B-1 : une séquence d'acides aminés représentée par la SÉQ ID N° 3
Séquence B-2 : une séquence d'acides aminés qui partage l'identité d'égale ou supérieure à 80 % avec la séquence d'acides aminés représentée par la SÉQ ID N° 3 et est capable de se fixer au récipient de culture
Séquence B-3 : une séquence d'acides aminés qui est obtenue par addition, substitution ou délétion d'un ou plusieurs résidus d'acides aminés dans la séquence d'acides aminés représentée par la SÉQ ID N° 3 et est capable de se fixer au récipient de culture.

10. Récipient de culture selon la revendication 9,
dans lequel le polypeptide est dans un état sec, ou
dans lequel le polypeptide est dans un état humide dans lequel un liquide qui ne contient sensiblement pas le facteur de croissance fibroblastique basique est retenu.

11. Récipient de culture selon l'une quelconque des revendications 9 ou 10,
dans lequel le polypeptide inclut en outre un motif RGD.

12. Matériau de support destiné à la mise en culture de cellules, comprenant :
un polypeptide qui présente une activité d'adhérence cellulaire et inclut un premier domaine destiné à lier un facteur de croissance fibroblastique basique à un second domaine pour se fixer à un récipient de culture,
dans lequel le facteur de croissance fibroblastique basique est lié au premier domaine,
dans lequel le nombre de résidus d'acides aminés du polypeptide va de 80 à 250,
dans lequel le premier domaine inclut l'une quelconque de la séquence A-1, la séquence A-2 et la séquence A-3 suivantes, et
le second domaine inclut l'une quelconque de la séquence B-1, la séquence B-2 et la séquence B-3 suivantes ;
Séquence A-1 : une séquence d'acides aminés représentée par la SÉQ ID N° 2
Séquence A-2 : une séquence d'acides aminés qui partage l'identité d'égale ou supérieure à 80 % avec la séquence d'acides aminés représentée par la SÉQ ID N° 2 et est capable de se lier au facteur de croissance fibroblastique basique
Séquence A-3 : une séquence d'acides aminés qui est obtenue par addition, substitution ou délétion d'un ou plusieurs résidus d'acides aminés dans la séquence d'acides aminés représentée par la SÉQ ID N° 2 et est capable de se lier au facteur de croissance fibroblastique basique
Séquence B-1 : une séquence d'acides aminés représentée par la SÉQ ID N° 3
Séquence B-2 : une séquence d'acides aminés qui partage l'identité d'égale ou supérieure à 80 % avec la séquence d'acides aminés représentée par la SÉQ ID N° 3 et est capable de se fixer au récipient de culture
Séquence B-3 : une séquence d'acides aminés qui est obtenue par addition, substitution ou délétion d'un ou plusieurs résidus d'acides aminés dans la séquence d'acides aminés représentée par la SÉQ ID N° 3 et est capable de se fixer au récipient de culture.

13. Matériau de support destiné à la mise en culture de cellules selon la revendication 12,
qui est dans un état sec, ou
qui est dans un état humide dans lequel un liquide qui ne contient pas sensiblement le facteur de croissance fibroblastique basique est retenu.

14. Matériau de support destiné à la mise en culture de cellules selon l'une quelconque des revendications 12 à 13,
dans lequel le polypeptide inclut en outre un motif RGD.


### SEQUENCE LISTING

<110> FUJIFILM CORPORATION
<120> Method for Culturing Multipotent Stem Cells
<130> FS-F06376-00
<150> JP 2015-159967
   <151> 2015-08-13
<160> 16
<170> PatentIn version 3.5
<210> 1
   <211> 459
   <212> PRT
   <213> Homo sapiens
<400> 1
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Tyr Thr Val Tyr Asp Asp Gly Glu Glu
       50                  55                  60
   Lys Asn Asn Ala Thr Val His Glu Gln Val Gly Gly Pro Ser Leu Thr
   65                  70                  75                  80
   Ser Asp Leu Gln Ala Gln Ser Lys Gly Asn Pro Glu Gln Thr Pro Val
                   85                  90                  95
   Leu Lys Pro Glu Glu Glu Ala Pro Ala Pro Glu Val Gly Ala Ser Lys
               100                 105                 110
   Pro Glu Gly Ile Asp Ser Arg Pro Glu Thr Leu His Pro Gly Arg Pro
           115                 120                 125
   Gln Pro Pro Ala Glu Glu Glu Leu Cys Ser Gly Lys Pro Phe Asp Ala
       130                 135                 140
   Phe Thr Asp Leu Lys Asn Gly Ser Leu Phe Ala Phe Arg Gly Gln Tyr
   145                 150                 155                 160
   Cys Tyr Glu Leu Asp Glu Lys Ala Val Arg Pro Gly Tyr Pro Lys Leu
   165 170 175
   Ile Arg Asp Val Trp Gly Ile Glu Gly Pro Ile Asp Ala Ala Phe Thr
   180 185 190
   Arg Ile Asn Cys Gln Gly Lys Thr Tyr Leu Phe Lys Gly Ser Gln Tyr
   195 200 205
   Trp Arg Phe Glu Asp Gly Val Leu Asp Pro Asp Tyr Pro Arg Asn Ile
   210 215 220
   Ser Asp Gly Phe Asp Gly Ile Pro Asp Asn Val Asp Ala Ala Leu Ala
   225 230 235 240
   Leu Pro Ala His Ser Tyr Ser Gly Arg Glu Arg Val Tyr Phe Phe Lys
   245 250 255
   Gly Lys Gln Tyr Trp Glu Tyr Gln Phe Gln His Gln Pro Ser Gln Glu
   260 265 270
   Glu Cys Glu Gly Ser Ser Leu Ser Ala Val Phe Glu His Phe Ala Met
   275 280 285
   Met Gln Arg Asp Ser Trp Glu Asp Ile Phe Glu Leu Leu Phe Trp Gly
   290 295 300
   Arg Thr Ser Ala Gly Thr Arg Gln Pro Gln Phe Ile Ser Arg Asp Trp
   305 310 315 320
   His Gly Val Pro Gly Gln Val Asp Ala Ala Met Ala Gly Arg Ile Tyr
   325 330 335
   Ile Ser Gly Met Ala Pro Arg Pro Ser Leu Ala Lys Lys Gln Arg Phe
   340 345 350
   Arg His Arg Asn Arg Lys Gly Tyr Arg Ser Gln Arg Gly His Ser Arg
   355 360 365
   Gly Arg Asn Gln Asn Ser Arg Arg Pro Ser Arg Ala Thr Trp Leu Ser
   370 375 380
   Leu Phe Ser Ser Glu Glu Ser Asn Leu Gly Ala Asn Asn Tyr Asp Asp
   385 390 395 400
   Tyr Arg Met Asp Trp Leu Val Pro Ala Thr Cys Glu Pro Ile Gln Ser
   405 410 415
   Val Phe Phe Phe Ser Gly Asp Lys Tyr Tyr Arg Val Asn Leu Arg Thr
               420                 425                 430
   Arg Arg Val Asp Thr Val Asp Pro Pro Tyr Pro Arg Ser Ile Ala Gln
           435                 440                 445
   Tyr Trp Leu Gly Cys Pro Ala Pro Gly His Leu
       450                 455
<210> 2
   <211> 42
   <212> PRT
   <213> Artificial Sequence
<220>
   <223> SMB domain
<400> 2
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln
           35                  40
<210> 3
   <211> 32
   <212> PRT
   <213> Artificial Sequence
<220>
   <223> Heparin Binding Domain
<400> 3
   Pro Arg Pro Ser Leu Ala Lys Lys Gln Arg Phe Arg His Arg Asn Arg
   1               5                   10                  15
   Lys Gly Tyr Arg Ser Gln Arg Gly His Ser Arg Gly Arg Asn Gln Asn
               20                  25                  30
<210> 4
   <211> 55
   <212> PRT
   <213> Artificial Sequence
<220>
   <223> SMB domain and RGD motif
<400> 4
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu
       50                  55
<210> 5
   <211> 87
   <212> PRT
   <213> Artificial Sequence
<220>
   <223> No.5
<400> 5
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Pro Arg Pro Ser Leu Ala Lys Lys Gln
       50                  55                  60
   Arg Phe Arg His Arg Asn Arg Lys Gly Tyr Arg Ser Gln Arg Gly His
   65                  70                  75                  80
   Ser Arg Gly Arg Asn Gln Asn
                   85
<210> 6
   <211> 107
   <212> PRT
   <213> Artificial Sequence
<220>
   <223> No.6
<400> 6
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Gly Val Pro Gly Gln Val Asp Ala Ala
       50                  55                  60
   Met Ala Gly Arg Ile Tyr Ile Ser Gly Met Ala Pro Arg Pro Ser Leu
   65                  70                  75                  80
   Ala Lys Lys Gln Arg Phe Arg His Arg Asn Arg Lys Gly Tyr Arg Ser
                   85                  90                  95
   Gln Arg Gly His Ser Arg Gly Arg Asn Gln Asn
               100                 105
<210> 7
   <211> 117
   <212> PRT
   <213> Artificial Sequence
<220>
   <223> No.7
<400> 7
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Gln Pro Gln Phe Ile Ser Arg Asp Trp
       50                  55                  60
   His Gly Val Pro Gly Gln Val Asp Ala Ala Met Ala Gly Arg Ile Tyr
   65                  70                  75                  80
   Ile Ser Gly Met Ala Pro Arg Pro Ser Leu Ala Lys Lys Gln Arg Phe
                   85                  90                  95
   Arg His Arg Asn Arg Lys Gly Tyr Arg Ser Gln Arg Gly His Ser Arg
               100                 105                 110
   Gly Arg Asn Gln Asn
                               115
<210> 8
   <211> 127
   <212> PRT
   <213> Artificial Sequence
<220>
   <223> No.8
<400> 8
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Phe Trp Gly Arg Thr Ser Ala Gly Thr
       50                  55                  60
   Arg Gln Pro Gln Phe Ile Ser Arg Asp Trp His Gly Val Pro Gly Gln
   65                  70                  75                  80
   Val Asp Ala Ala Met Ala Gly Arg Ile Tyr Ile Ser Gly Met Ala Pro
                   85                  90                  95
   Arg Pro Ser Leu Ala Lys Lys Gln Arg Phe Arg His Arg Asn Arg Lys
               100                 105                 110
   Gly Tyr Arg Ser Gln Arg Gly His Ser Arg Gly Arg Asn Gln Asn
           115                 120                 125
<210> 9
   <211> 142
   <212> PRT
   <213> Artificial Sequence
<220>
   <223> No.9
<400> 9
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
   35 40 45
   Phe Thr Met Pro Glu Asp Glu Ser Gln Glu Glu Ser Glu Gly Ser Glu
   50 55 60
   Asp Ile Phe Glu Leu Leu Phe Trp Gly Arg Thr Ser Ala Gly Thr Arg
   65 70 75 80
   Gln Pro Gln Phe Ile Ser Arg Asp Trp His Gly Val Pro Gly Gln Val
   85 90 95
   Asp Ala Ala Met Ala Gly Arg Ile Tyr Ile Ser Gly Met Ala Pro Arg
   100 105 110
   Pro Ser Leu Ala Lys Lys Gln Arg Phe Arg His Arg Asn Arg Lys Gly
   115 120 125
   Tyr Arg Ser Gln Arg Gly His Ser Arg Gly Arg Asn Gln Asn
   130 135 140
<210> 10
   <211> 159
   <212> PRT
   <213> Artificial Sequence
<220>
   <223> No.10
<400> 10
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Ser
       50                  55                  60
   Leu Ser Ala Val Phe Glu His Phe Ala Met Met Gln Arg Asp Ser Trp
   65                  70                  75                  80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
       130                 135                 140
   Gly Tyr Arg Ser Gln Arg Gly His Ser Arg Gly Arg Asn Gln Asn
   145                 150                 155
<210> 11
   <211> 165
   <212> PRT
   <213> Artificial Sequence
<220>
   <223> No.11
<400> 11
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Ser
       50                  55                  60
   Leu Ser Ala Val Phe Glu His Phe Ala Met Met Gln Arg Asp Ser Trp
   65                  70                  75                  80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
       130                 135                 140
   Gly Tyr Arg Ser Gln Arg Gly His Ser Arg Gly Arg Asn Gln Asn Ser
   145                 150                 155                 160
   Arg Arg Pro Ser Arg
                   165
<210> 12
   <211> 175
   <212> PRT
   <213> Artificial Sequence
<220>
   <223> No.12
<400> 12
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Ser
       50                  55                  60
   Leu Ser Ala Val Phe Glu His Phe Ala Met Met Gln Arg Asp Ser Trp
   65                  70                  75                  80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
       130                 135                 140
   Gly Tyr Arg Ser Gln Arg Gly His Ser Arg Gly Arg Asn Gln Asn Ser
   145                 150                 155                 160
   Arg Arg Pro Ser Arg Ala Thr Trp Leu Ser Leu Phe Ser Ser Glu
                   165                 170                 175
<210> 13
   <211> 185
   <212> PRT
   <213> Artificial Sequence
<220>
   <223> No.13
<400> 13
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Ser
       50                  55                  60
   Leu Ser Ala Val Phe Glu His Phe Ala Met Met Gln Arg Asp Ser Trp
   65                  70                  75                  80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
       130                 135                 140
   Gly Tyr Arg Ser Gln Arg Gly His Ser Arg Gly Arg Asn Gln Asn Ser
   145                 150                 155                 160
   Arg Arg Pro Ser Arg Ala Thr Trp Leu Ser Leu Phe Ser Ser Glu Glu
                   165                 170                 175
   Ser Asn Leu Gly Ala Asn Asn Tyr Asp
               180                 185
<210> 14
   <211> 245
   <212> PRT
   <213> Artificial Sequence
<220>
   <223> No.14
<400> 14
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Ser Gln Glu Glu Ser Glu Gly Ser Ser
       50                  55                  60
   Leu Ser Ala Val Phe Glu His Phe Ala Met Met Gln Arg Asp Ser Trp
   65                  70                  75                  80
   Glu Asp Ile Phe Glu Leu Leu Phe Trp Gly Arg Thr Ser Ala Gly Thr
                   85                  90                  95
   Arg Gln Pro Gln Phe Ile Ser Arg Asp Trp His Gly Val Pro Gly Gln
               100                 105                 110
   Val Asp Ala Ala Met Ala Gly Arg Ile Tyr Ile Ser Gly Met Ala Pro
           115                 120                 125
   Arg Pro Ser Leu Ala Lys Lys Gln Arg Phe Arg His Arg Asn Arg Lys
       130                 135                 140
   Gly Tyr Arg Ser Gln Arg Gly His Ser Arg Gly Arg Asn Gln Asn Ser
   145                 150                 155                 160
   Arg Arg Pro Ser Arg Ala Thr Trp Leu Ser Leu Phe Ser Ser Glu Glu
                   165                 170                 175
   Ser Asn Leu Gly Ala Asn Asn Tyr Asp Asp Tyr Arg Met Asp Trp Leu
               180                 185                 190
   Val Pro Ala Thr Ser Glu Pro Ile Gln Ser Val Phe Phe Phe Ser Gly
           195                 200                 205
   Asp Lys Tyr Tyr Arg Val Asn Leu Arg Thr Arg Arg Val Asp Thr Val
       210                 215                 220
   Asp Pro Pro Tyr Pro Arg Ser Ile Ala Gln Tyr Trp Leu Gly Ser Pro
   225                 230                 235                 240
   Ala Pro Gly His Leu
   245
<210> 15
   <211> 246
   <212> PRT
   <213> Artificial Sequence
<220>
   <223> No.15
<400> 15
   Asp Gln Glu Ser Cys Lys Gly Arg Cys Thr Glu Gly Phe Asn Val Asp
   1               5                   10                  15
   Lys Lys Cys Gln Cys Asp Glu Leu Cys Ser Tyr Tyr Gln Ser Cys Cys
               20                  25                  30
   Thr Asp Tyr Thr Ala Glu Cys Lys Pro Gln Val Thr Arg Gly Asp Val
           35                  40                  45
   Phe Thr Met Pro Glu Asp Glu Pro Ser Gln Glu Glu Cys Glu Gly Ser
       50                  55                  60
   Ser Leu Ser Ala Val Phe Glu His Phe Ala Met Met Gln Arg Asp Ser
   65                  70                  75                  80
   Trp Glu Asp Ile Phe Glu Leu Leu Phe Trp Gly Arg Thr Ser Ala Gly
                   85                  90                  95
   Thr Arg Gln Pro Gln Phe Ile Ser Arg Asp Trp His Gly Val Pro Gly
               100                 105                 110
   Gln Val Asp Ala Ala Met Ala Gly Arg Ile Tyr Ile Ser Gly Met Ala
           115                 120                 125
   Pro Arg Pro Ser Leu Ala Lys Lys Gln Arg Phe Arg His Arg Asn Arg
       130                 135                 140
   Lys Gly Tyr Arg Ser Gln Arg Gly His Ser Arg Gly Arg Asn Gln Asn
   145                 150                 155                 160
   Ser Arg Arg Pro Ser Arg Ala Thr Trp Leu Ser Leu Phe Ser Ser Glu
                   165                 170                 175
   Glu Ser Asn Leu Gly Ala Asn Asn Tyr Asp Asp Tyr Arg Met Asp Trp
               180                 185                 190
   Leu Val Pro Ala Thr Cys Glu Pro Ile Gln Ser Val Phe Phe Phe Ser
           195                 200                 205
   Gly Asp Lys Tyr Tyr Arg Val Asn Leu Arg Thr Arg Arg Val Asp Thr
       210                 215                 220
   Val Asp Pro Pro Tyr Pro Arg Ser Ile Ala Gln Tyr Trp Leu Gly Cys
   225                 230                 235                 240
   Pro Ala Pro Gly His Leu
                   245
<210> 16
   <211> 143
   <212> PRT
   <213> Artificial Sequence
<220>
   <223> RCP-1
<400> 16
   Met Asp Gln Glu Ser Cys Lys Gly Arg Cys Thr Glu Gly Phe Asn Val
   1               5                   10                  15
   Asp Lys Lys Cys Gln Cys Asp Glu Leu Cys Ser Tyr Tyr Gln Ser Cys
               20                  25                  30
   Cys Thr Asp Tyr Thr Ala Glu Cys Lys Pro Gln Val Thr Arg Gly Asp
           35                  40                  45
   Val Phe Thr Met Pro Glu Asp Glu Ser Gln Glu Glu Ser Glu Gly Ser
       50                  55                  60
   Glu Asp Ile Phe Glu Leu Leu Phe Trp Gly Arg Thr Ser Ala Gly Thr
   65                  70                  75                  80
   Arg Gln Pro Gln Phe Ile Ser Arg Asp Trp His Gly Val Pro Gly Gln
                   85                  90                  95
   Val Asp Ala Ala Met Ala Gly Arg Ile Tyr Ile Ser Gly Met Ala Pro
               100                 105                 110
   Arg Pro Ser Leu Ala Lys Lys Gln Arg Phe Arg His Arg Asn Arg Lys
           115                 120                 125
   Gly Tyr Arg Ser Gln Arg Gly His Ser Arg Gly Arg Asn Gln Asn
       130                 135                 140